# EUROPEAN PATENT SPECIFICATION

(11) **EP 4 164 629 B1**
(45) Date of publication and mention of the grant of the patent: **05.06.2024**
(21) Application number: 21731159.6
(22) Date of filing: 09.06.2021
(51) Int. Cl.: A61K 31/353, A61P 31/12, A61K 36/15

(54) **PROCYANIDINS FOR THE TREATMENT OF ENDOTHELIAL DYSFUNCTION TRIGGERED BY COVID-19**
PROCYANIDINE ZUR BEHANDLUNG VON ENDOTHELIALEN ENTZÜNDUNGEN BEI COVID-19-PATIENTEN
PROCYANIDINES POUR LE TRAITEMENT DE L'INFLAMMATION ENDOTHÉLIALE CHEZ DES PATIENTS COVID-19

(30) Priority: 18.06.2020 EP 20180939
(43) Date of publication of application: 19.04.2023
(73) Proprietor: Horphag Research IP (Pyc) Ltd., 3032 Limassol (CY)
(72) Inventor: FERRARI, Victor, 1216 Cointrin (CH); BURKI, Carolina, 1216 Cointrin (CH); WEICHMANN, Franziska, 1216 Cointrin (CH)
(74) Representative: AWA Switzerland
(86) International application number: PCT/EP2021/065482
(87) International publication number: WO 2021/254851

(56) References cited:
- JP-A- 2005 314 316
- JP-A- 2007 217 410
- ZHUANG M ET AL: "Procyanidins and butanol extract of Cinnamomi Cortex inhibit SARS-CoV infection", ANTIVIRAL RESEARCH, ELSEVIER BV, NL, vol. 82, no. 1, 1 April 2009 (2009-04-01), pages 73-81, XP025981716, ISSN: 0166-3542, DOI: 10.1016/J.ANTIVIRAL.2009.02.001 [retrieved on 2009-02-11]
- ISTIFLI ERMAN SALIH ET AL: "In silicoanalysis of the interactions of certain flavonoids with the receptor-binding domain of 2019 novel coronavirus and cellular proteases and their pharmacokinetic properties", JOURNAL OF BIOMOLECULAR STRUCTURE & DYNAMICS DEC 2013, , vol. ahead-of-print, no. ahead-of-print 28 October 2020 (2020-10-28), pages 1-15, XP009525294, ISSN: 1538-0254, DOI: 10.1080/07391102.2020.1840444 Retrieved from the Internet: URL:https://www.tandfonline.com/doi/full/1 0.1080/07391102.2020.1840444 [retrieved on 2020-10-28]
- MAEDA TAKAAKI ET AL: "Anti SARS-CoV Activity of Extracts from Japanese Pepper (Zanthoxylum piperitum (L.) DC. f. inerme Makino)", HORTICULTURAL RESEARCH (JAPAN), ????, JP , vol. 10, no. 2 15 April 2011 (2011-04-15), pages 267-272, XP009525292, ISSN: 1347-2658, DOI: 10.2503/HRJ.10.267 Retrieved from the Internet: URL:http://joi.jlc.jst.go.jp/JST.JSTAGE/hr j/10.267?from=CrossRef
- ROH CHANGHYUN: "A facile inhibitor screening of SARS coronavirus N protein using nanoparticle-based RNA oligonucleotide", INTERNATIONAL JOURNAL OF NANOMEDICINE, DOVE MEDICAL PRESS, NEW ZEALAND , vol. 7 1 January 2012 (2012-01-01), pages 2173-2179, XP009525291, ISSN: 1178-2013, DOI: 10.2147/IJN.S31379 Retrieved from the Internet: URL:http://www.dovepress.com/a-facile-inhi bitor-screening-of-sars-coronavirus-n-prot ein-using-nanop-peer-reviewed-article-IJN [retrieved on 2012-05-01]
- SALMAN SAAD ET AL: "Virtual screening of immunomodulatory medicinal compounds as promising anti-SARS-CoV-2 inhibitors", FUTURE VIROLOGY, FUTURE MEDICINE LTD., UK , vol. 15, no. 5 30 April 2020 (2020-04-30), pages 267-275, XP009525290, ISSN: 1746-0794, DOI: 10.2217/FVL-2020-0079 Retrieved from the Internet: URL:https://www.futuremedicine.com/doi/10. 2217/fvl-2020-0079

## Description

### FIELD OF THE INVENTION

The invention relates to a natural composition for medical purposes and more specifically to a composition comprising procyanidins, for use in the prevention or treatment of endothelial inflammation and/or endothelial systemic dysfunction triggered by Corona virus disease 2019 (COVID-19) including symptomatic post-COVID-19 subjects recovering from COVID-19.

### BACKGROUND OF THE INVENTION

Coronavirus disease 2019 (COVID-19) is defined as illness caused by a novel coronavirus now called severe acute respiratory syndrome coronavirus 2 (SARS-CoV-2; formerly called 2019-nCoV), which was first identified amid an outbreak of respiratory illness cases in Wuhan City, Hubei Province, China. It was initially reported to the WHO on December 31, 2019. On January 30, 2020, the WHO declared the COVID-19 outbreak a global health emergency. On March 11, 2020, the WHO declared COVID-19 a global pandemic, its first such designation since declaring H1N1 influenza a pandemic in 2009. As of 9 June 2020, more than 7.12 million cases have been reported across 188 countries and territories, resulting in more than 406,000 deaths.

Common symptoms include fever, cough, fatigue, shortness of breath, and loss of smell and taste. While the majority of cases result in mild symptoms, some progress to acute respiratory distress syndrome (ARDS) likely precipitated by a cytokine storm, multi-organ failure, septic shock, and blood clots.

Complications may include pneumonia, acute respiratory distress syndrome (ARDS), multi-organ failure, septic shock, and death. Cardiovascular complications may include heart failure, arrhythmias, heart inflammation, and blood clots. Approximately 20-30% of people who present with COVID-19 have elevated liver enzymes reflecting liver injury. Neurologic manifestations include seizure, stroke, encephalitis, and Guillain-Barré syndrome (which includes loss of motor functions). Following the infection, children may develop paediatric multisystem inflammatory syndrome, which has symptoms similar to Kawasaki disease, which can be fatal.

Varga Zsuzsanna et al. "Endothelial cell infection and endotheliitis in COVID-19" www.thelancet.com, Vol 395, May 2, 2020, describe that cardiovascular complications are rapidly emerging as a key threat in coronavirus disease 2019 (COVID-19) in addition to respiratory disease. The mechanisms underlying the disproportionate effect of severe acute respiratory syndrome coronavirus 2 (SARS-CoV-2) infection on patients with cardiovascular comorbidities, however, remain incompletely understood. SARS-CoV-2 infects the host using the angiotensin converting enzyme 2 (ACE2) receptor, which is expressed in several organs, including the lung, heart, kidney, and intestine. ACE2 receptors are also expressed by endothelial cells. Whether vascular derangements in COVID-19 are due to endothelial cell involvement by the virus is currently unknown. Intriguingly, SARS-CoV-2 can directly infect engineered human blood vessel organoids in vitro. Indeed, it was demonstrated endothelial cell involvement across vascular beds of different organs in a series of patients with COVID-19. The authors found evidence of direct viral infection of the endothelial cell and diffuse endothelial inflammation. Recruitment of immune cells, either by direct viral infection of the endothelium or immune-mediated, can result in widespread endothelial dysfunction associated with apoptosis. These findings show the presence of viral elements within endothelial cells and an accumulation of inflammatory cells, with evidence of endothelial and inflammatory cell death. This also suggests that SARS-CoV-2 infection facilitates the induction of endotheliitis in several organs as a direct consequence of viral involvement and of the host inflammatory response. In addition, induction of apoptosis and pyroptosis might have an important role in endothelial cell injury in patients with COVID-19. COVID-19-endotheliitis could explain the systemic impaired microcirculatory function in different vascular beds and their clinical sequelae in patients with COVID-19.

This was also confirmed by Frank Ruschitzka et al. in The Lancet, "Endothelial cell infection and endotheliitis in COVID-19" April 20, 2020. COVID-19 was considered to be a lung disease. Up until now, it has been unclear as to why patients are sustaining life-threatening organ failure in organs other than the lungs. An interdisciplinary team from University Hospital Zurich has now shown that SARS-CoV-2 directly elicits inflammation in blood vessels and that this can lead to organ failure and even death. During analyses of tissue samples taken from deceased COVID-19 patients taken following an autopsy, pathologists at University Hospital Zurich have discovered that patients are not just suffering from an inflammation of the lungs, but also from an inflammation of all endothelial tissue in a wide range of organs. COVID is a systemic inflammation of the blood vessels and we may now also refer to the disease as COVID-Endotheliitis," said Prof. Frank Ruschitzka, summarizing the findings to which cardiologists, infectiologists, pathologists and intensive care physicians have contributed. Frank Ruschitzka also believes that treatment for COVID-19 patients must address two points: "We have to tackle viruses' replication and protecting and stabilizing patients' vascular systems at the same time. This applies primarily to patients suffering from cardiovascular diseases and have already been diagnosed as having an impaired endothelial function, as well as to those of our patients with known risk factors for a severe progression of COVID-19."

Zuhang et al. "Procyanidins and butanol extract of Cinnamomi Cortex inhibit SARS-CoV infection" Antiviral Res. 2009 Apr; 82(1): 73-81, Published online 2009 Feb 11. doi: 10.1016/j.antiviral.2009.02.001, found that the butanol fraction of Cinnamomi Cortex (CC/Fr.2) showed moderate inhibitory activity in wild-type severe acute respiratory syndrome coronavirus (wtSARS-CoV) and HIV/SARS-CoV S pseudovirus infections. The inhibition on pseudovirus was also seen in cells pre-treated with the CC and CC/Fr.2 (IC_{50S}, 283.4 ± 16.3 and 149.5 ± 13.5 µg/ml, respectively), however the highest activities on wtSARS-CoV were observed when the viruses were treated by the extracts before challenging (IC_{50S}, 43.1 ± 2.8 and 7.8 ± 0.3 µg/ml; SIs, 8.4 and 23.1, respectively). Among the compounds fractionated from CC, procyanidin A2 and procyanidin B1 showed moderate anti-wtSARS-CoV activity (IC_{50S}, 29.9 ± 3.3 and 41.3 ± 3.4 µM; SIs, 37.35 and 15.69, respectively). Authors also sought to determine whether they could interfere with the clathrin-dependent endocytosis pathway using transferrin receptor (TfR) as an indicator. CC/Fr.2 inhibited the internalization of TfR but the procyanidins did not. Taken together, CC/Fr.2 contains unknown substances, that could inhibit the infection, probably by interfering with endocytosis, and it also contains procyanidins that did not inhibit the internalization but inhibited the infection. Therefore, CC extracts contain anti-virus activities that act through distinct mechanisms according to differences in the compounds or mixtures.

JP 2005 314316 A (Kikkoman Corp.) provides (1) a new anti-SARS coronavirus agent containing proanthocyanidin, catechin or a grape extract as an active component, (2) an agent for the prevention or treatment of SARS coronavirus infection diseases containing proanthocyanidin, catechin or the grape extract as an active component, (3) a food, drink, medicine or cosmetic containing the above anti-SARS coronavirus agent or an agent for the prevention or treatment of SARS coronavirus infection diseases and (4) a food or drink containing proanthocyanidin, catechin or the grape extract as an active component and labelled to be used for the prevention or amelioration of SARS coronavirus infection diseases.

JP 2007 217410 A (Yoshida Tsutomu, Yamashita Masako) provides an antiviral composition eliminating a virus from a virus latent infection cell, and contributing to the fundamental therapy of viral infectious disease; and also provides an antiviral agent containing the composition as active ingredients, and an antiviral functional food. The antiviral agent has virus proliferation-inhibiting activities by containing fucoidan or proanthocyanidin as an active ingredient against the virus latent infection cell, or virus-inducing activities from the virus latent infection cell by containing the fucoidan or the proanthocyanidin as an active ingredient. The medicine for treating the virus or the antiviral functional food is obtained by containing the antiviral composition. Especially preferably, the fucoidan is a sulfated polysaccharide derived from brown algae, and the proanthocyanidin is the one contained in an extract of peanut seed coat.

ISTIFLI ERMAN SALIH et al.: "In silico analysis of the interactions of certain flavonoids with the receptor-binding domain of 2019 novel coronavirus and cellular proteases and their pharmacokinetic properties", JOURNAL OF BIOMOLECULAR STRUCTURE & DYNAMICS DEC 2013, vol. ahead-of-print, no. ahead-of-print 28 October 2020 (2020-10-28), pages 1-15, XP009525294, discloses that Coronavirus Disease 2019 (COVID-19) has infected more than thirty five million people worldwide and caused nearly 1 million deaths as of October 2020. The microorganism causing COVID-19 was named as Severe Acute Respiratory Syndrome Coronavirus 2 (SARS-CoV-2 or 2019-nCoV). The aim of this study was to investigate the interactions of twenty-three phytochemicals belonging to different flavonoid subgroups with the receptor binding domain (RBD) of the spike glycoprotein of 2019-nCoV, and cellular proteases [transmembrane serine protease 2 (TMPRSS2), cathepsin B and L (CatB/L)]. The compounds interacted more strongly with CatB and CatL than with the other proteins. Van der Waals and hydrogen bonds played an important role in the receptor-ligand interactions. As a result of RBCI (relative binding capacity index) analysis conducted to rank flavonoids in terms of their interactions with the target proteins, (-)-epicatechin gallate interacted strongly with all the proteins studied. The results obtained from molecular dynamics and molecular mechanics Poisson-Boltzmann surface area (MM/PBSA) methods also supported this data. According to Lipinski's rule of five, (-)-epicatechin gallate showed drug-likeness properties. Although this molecule is not capable of crossing the blood-brain barrier (BBB), it was concluded that (-)-epicatechin gallate can be evaluated as a candidate molecule in drug development studies against 2019-nCoV since it was not the substrate of P-gp (P-glycoprotein), did not inhibit any of the cytochrome Ps, and did not show AMES toxicity or hepatotoxicity on eukaryotic cells.

MAEDA TAKAAKI ET al: "Anti SARS-CoV Activity of Extracts from Japanese Pepper (Zanthoxylum piperitum (L.) DC. f. inerme Maki no)", HORTICULTURAL RESEARCH (JAPAN), JP vol. 10, no. 2, 15 April 2011 (2011-04-15), pages 267-272, XP009525292, discloses that Japanese pepper, *Zanthoxylum piperitum,* is native to Japan and has four well-known lineages (Asakura, Takahara, Budou, and Arima), which are named after their production area or morphology. Restriction-site associated DNA sequencing (RAD-Seq) was used to analyse 93 accessions from various areas, including these four lineages. Single nucleotide variant analysis was used to classify the plants into eight groups: the Asakura and Arima lineages each had two groups, the Takahara and Budou lineages each had one group, and two additional groups were present. In one Asakura group and two Arima groups, the plants were present in agricultural fields and mountains, thus representing the early stage of domestication of the Japanese pepper. The second Asakura lineage group was closely related to plants present in various areas, and this represents the second stage of domestication of this plant because, after early domestication, genetically related lineages with desirable traits spread to the periphery. These results demonstrate that domestication of Japanese pepper is ongoing. In addition, this study shows that spineless plants are polyphyletic, despite the spineless lineage being considered a subspecies of Japanese pepper.

ROH CHANGHYUN et al.: "A facile inhibitor screening of SARS coronavirus N protein using nanoparticle-based RNA oligonucleotide", INTERNATIONAL JOURNAL OF NANOMEDICINE, DOVE MEDICAL PRESS, NEW ZEALAND, Vol. 7, 1 January 2012 (2012-01-01), pages 2173-2179, XP009525291, discloses that hundreds of million people worldwide have been infected with severe acute respiratory syndrome (SARS), and the rate of global death from SARS has remarkably increased. Hence, the development of efficient drug treatments for the biological effects of SARS is highly needed. Authors have previously shown that quantum dots (QDs)-conjugated RNA oligonucleotide is sensitive to the specific recognition of the SARS-associated coronavirus (SARS-CoV) nucleocapsid (N) protein. In this study, Authors found that a designed biochip could analyze inhibitors of the SARS-CoV N protein using nanoparticle-based RNA oligonucleotide. Among the polyphenolic compounds examined, (-)-catechin gallate and (-)-gallocatechin gallate demonstrated a remarkable inhibition activity on SARS-CoV N protein. (-)-catechin gallate and (-)-gallocatechin gallate attenuated the binding affinity in a concentrated manner as evidenced by QDs-conjugated RNA oligonucleotide on a designed biochip. At a concentration of 0.05 µg mL-1, (-)-catechin gallate and (-)-gallocatechin gallate showed more than 40% inhibition activity on a nanoparticle-based RNA oligonucleotide biochip system.

SALMAN SAAD ET AL: "Virtual screening of immunomodulatory medicinal compounds as promising anti-SARS-CoV-2 inhibitors", FUTURE VIROLOGY, FUTURE MEDICINE LTD., UK, Vol. 15, no. 5, 30 April 2020 (2020-04-30), pages 267-275, XP009525290, discloses that Severe acute respiratory syndrome coronavirus-2 (SARS-CoV-2), a pernicious viral disease, causes acute respiratory distress responsible for mortality and morbidity worldwide. To screen different immunomodulatory medicinal compounds to unravel their interaction with SARS-CoV-2 viral proteins. Materials & methods: A library of immunomodulatory medicinal compounds with antiviral capability were analyzed against SARS proteases, spike protein and nonstructural proteins (NSP-9, 15) using Autodock vina. Results: Out of more than 300 medicinal compounds, only six compounds: arzanol, ferulic acid, genistein, resveratrol, rosmanol and thymohydroquinone showed significant interaction with the SARS viral proteins by forming hydrogen bonds with the active site residues with low binding energy. Further ADMET (absorption, distribution, metabolism, excretion and toxicity) analysis showed good pharmacokinetic properties and low acute toxicity of these compounds. Conclusion: The current study provides convincing evidence that these medicinal compounds exert antiviral activity against the SARS-CoV-2 virus and could be further exploited for the treatment of this disease.

All these documents disclose in silica studies.

In particular, the cited documents Zuhang et al., JP 2005 314316 A, JP 2007 217410 A, MAEDA TAKAAKI ET al and ROH CHANGHYUN et al describe anti-viral activities of proanthocyanidins derived from different sources by inhibiting the growth and proliferation of SARS-CoV / SARS-CoV2.

On the other hand, ISTIFLI ERMAN SALIH et al and SALMAN SAAD ET AL describe compounds, containing proanthocyanidins to have a high affinity or binding ability to proteins of SARS-CoV2, thereby also exerting anti-viral activity. An anti-viral activity of proanthocyanidins however is not the scope of the present invention and the existing prior art does therefore not apply in this context.

During the ongoing worldwide pandemic of severe acute respiratory syndrome coronavirus 2 (SARS-CoV-2), the number of patients recovering from coronavirus disease 2019 (COVID-19) is constantly growing. COVID-19 can result in a multi-organ disease with a broad spectrum of symptoms, ranging from pulmonary problems, thrombotic complications and cardiovascular dysfunctions, renal dysfunction, gastrointestinal symptoms, neurological problems, and many more. This pleiotropic clinical picture has been attributed to endothelial dysfunction, coagulopathy, microcirculatory and inflammatory problems. But, even after recovery from the acute COVID-19, there are many reports of persistent symptoms. A great responsibility now is to find and establish solutions for this growing worldwide problem of the post-COVID-19 condition.

After a SARS-CoV-2 infection, significant pulmonary symptoms may persist, particularly in subjects who have been admitted to the hospital and managed for at least 1 week on hospital wards (Nalbandian A, Sehgal K, Gupta A, Madhavan MV, McGroder C, Stevens JS, et al. Post-acute COVID-19 syndrome. Nature Medicine. 2021;27(4):601-15.).

Subjects admitted to intensive care units tend to have more important residual, morphologic, particularly respiratory damage because of the combination of disease, systemic complications, treatments and intubation.

But also COVID-19 patients with non-severe symptoms suffer from long-lasting effects such as fatigue, recurrent headaches, attention disorders, anxiety or depression and many more (Lopez-Leon S, Wegman-Ostrosky T, Perelman C, Sepulveda R, Rebolledo PA, Cuapio A, et al. More than 50 Long-term effects of COVID-19: a systematic review and meta-analysis. medRxiv. 2021:2021.01.27.21250617).

Permanent lung damage and scarring may also be seen after significant lung and respiratory involvement during viral infections. Post COVID-19 fibrosis is estimated to be prevalent in 1/3 of SARS-CoV-2 infected hospitalized patients ( Ahmad Alhiyari M, Ata F, Islam Alghizzawi M, Bint I Bilal A, Salih Abdulhadi A, Yousaf Z. Post COVID-19 fibrosis, an emerging complicationof SARS-CoV-2 infection. IDCases. 2020;23:e01041-e). The prolonged effects of the viral infection and their consequences render most patients symptomatic, weak, with sleep difficulties and unable to lead a normal life or work for a long period of time, often for more than six months.

Proanthocyanidins represent a group of plant polyphenols found in roots, barks and fruits with an astringent taste. Proanthocyanidins include the subgroups of procyanidins and prodelphinidins. Proanthocyanidins are biopolymers composed of flavan subunits. Procyanidins are composed of catechin and epicatechin units, also called monomeric procyanidins. Proanthocyanidins are extracted from plant material by conventional methods using solvents like water, ethanol or acetone or fluid carbon dioxide. The extracts are purified by solvent/solvent extraction, ultra-filtration or chromatographic procedures. The purified extracts are concentrated by solvent evaporation, freeze drying or spray drying.

A proanthocyanidin-rich extract from the bark of French maritime pine is distributed under the tradename Pycnogenol^{®} by Horphag Research. The extract contains 70-75% by weight procyanidins and other flavanols such as catechin, epicatechin and taxifolin, see Grimm et al. "Single and multiple dose pharmacokinetics of maritime pine bark extract (Pycnogenol) after oral administration to healthy volunteers" BMC Clinical Pharmacology, 03 August 2006, 6:4 - http://www.biomedcentral.com/1472-6904/6/4.

Other proanthocyanidins rich extracts can be obtained from grape seeds, cones from cypress trees, cocoa beans or other plant materials. Pycnogenol^{®} pine bark extract has been shown to stimulate endothelial nitric oxide synthase and to induce vasodilation (Fitzpatrick, D. F., Bing, B., Rohdewald, P., 1998).

US 2004137081 A1 (Rohdewald P. et al.) discloses that sexual wellness or sexual fitness is enhanced over time by administrating on a daily basis a source of proanthocyanidins and a source of arginine. Both sources may be blended into a composition or taken separately from a kit. The source of arginine may be a salt or peptide of L-arginine and aspartic acid such as arginine aspartate. The proanthocyanidins stimulate an endothelial NO-synthase enzyme, which serves as a catalyst for synthesis of the nitric oxide from a substrate that is the source of the arginine. A sufficient amount of the nitric oxide is released over time to enhance sexual wellness or sexual fitness. In case of low levels of androgenic hormones in both sexes, the combination may contain as a further ingredient a sex hormone or a sex hormone precursor or a sex hormone stimulant or a sex hormone bioavailability enhancer.

The vascular endothelium is an active paracrine, endocrine, and autocrine organ that is indispensable for the regulation of vascular tone and the maintenance of vascular homoeostasis. Endothelial dysfunction is a principal determinant of microvascular dysfunction by shifting the vascular equilibrium towards more vasoconstriction with subsequent organ ischaemia, inflammation with associated tissue oedema, and a procoagulant state.

Currently there is a rationale for therapies to stabilise the endothelium while tackling viral replication. This strategy could be particularly relevant for vulnerable patients with pre-existing endothelial dysfunction, which is associated with male sex, smoking, hypertension, diabetes, obesity, and established cardiovascular disease, all of which are associated with adverse outcomes in COVID-19.

Consequently, there is a need for an effective and safe natural composition for the treatment or the prevention of endothelial inflammation and/or endothelial systemic dysfunction triggered by Corona virus disease 2019 (COVID-19) including symptomatic post-COVID-19 subjects recovering from COVID-19.

### SUMMARY OF THE INVENTION

Applicants have surprisingly found that a composition comprising procyanidins shows an interesting potential in the prevention and/or treatment of endothelial inflammation and/or endothelial systemic dysfunction in patients infected by Severe Acute Respiratory Syndrome Corona Virus 2 (SARS-CoV-2). This safe natural composition is particularly promising in the treatment and prevention of endothelial inflammation and/or endothelial systemic dysfunction triggered by Corona virus disease 2019 (COVID-19).

Contrary to the prior art documents, the present invention solely aims at the treatment of endothelial dysfunction as a consequence of the Corona Virus Disease 2019 (COVID-19). No activity regarding anti-virality or any effects against the infection by SARS-CoV2 itself are made in the present invention, but the consequences of such an infection are addressed by the invention, thus applicants believe that the cited documents address different issues than those dealt with the present invention.

However, Weichmann, F. and Rohdewald, P., Projected supportive effects of Pycnogenol® in patients suffering from multi-dimensional health impairments after a SARS-CoV2 infection. Int J Antimicrob Agents, 2020. 56(6): p. 106191, discloses that SARS-CoV2 strongly affects endothelial cells, triggering an inflammation and/or coagulopathies and leading to microcirculatory dysfunction accompanying endothelial problems and pro-thrombotic conditions. The resulting symptoms of COVID-19 comprise endothelial dysfunction, coagulopathy, cytokine storm, microcirculation problems, and capillary leak syndrome. Data from previous studies with Pycnogenol^{®} offer good evidence for potential beneficial effects for patients suffering from COVID-19 by improving endothelial function and normalizing and stabilizing microcirculatory function and platelet activity and exerting anti-inflammatory and anti-oxidant effects.

It has been shown that the virus SARS-CoV2, responsible for COVID-19 strongly affects endothelial cells, leading to endothelial activation, pro-thrombotic conditions and microcirculatory dysfunction with increased inflammation and coagulopathies.

Procyanidins such as Pycnogenol^{®} showed to improve endothelial function by stimulation of the endothelial nitric oxide synthase (eNOS), which amplifies the NO generation and eventually leads to an increase in vessel lumen, adequate tissue perfusion and better blood circulation.

In addition, Pycnogenol^{®} has been shown to improve the microcirculation perfusion system. In several clinical studies the levels of O2 and CO2 in the tissues under the skin, the diameter of micro vessels in fingernails, as well as the blood flow velocity improved after Pycnogenol^{®} supplementation.

Furthermore, Pycnogenol^{®}-induced increased production of endothelial NO also leads to platelet activation, thereby lowering blood platelet aggregation, which lowers the risk of thrombosis, stroke or heart attack.

The potent anti-inflammatory activities of Pycnogenol^{®} were extensively investigated, showing a reduction of pro-inflammatory cytokine levels, such as COX-1 and 2, 5-LOX, TNF-α, IL-1β, IL-6 and NF-κB to normal levels.

During an inflammation, a number of reactive oxygen species are produced, which in turn fuel the inflammasome, leading to the secretion of interleukins. The antioxidant activity of Pycnogenol^{®} has been investigated in several clinical studies showing to both increase the plasma antioxidant capacity, expressed as oxygen radical absorbance capacity, and decrease the plasma oxidative stress measured as plasma free radicals.

In addition, the Pycnogenol^{®} metabolite M1 (δ-(3,4-dihydroxy-phenyl)-γ-valerolactone), which undergoes facilitated uptake by endothelial cells, was shown to exert direct anti-inflammatory activity by reducing iNOS (inducible nitric oxide synthase) expression and excessive nitrite production.

In one aspect of the present invention there is provided a composition comprising procyanidins according to the claims and at least one suitable excipient, for peroral use in the prevention or treatment of endothelial inflammation and/or endothelial systemic dysfunction triggered by Corona virus disease 2019 (COVID-19) induced by Severe Acute Respiratory Syndrome Corona Virus 2 (SARS-CoV-2) infection.

Preferably, the invention concerns a peroral composition comprising procyanidins originated from a plant extract selected from the group consisting in extracts of pine bark, grape seed, apples, cocoa, peanut skin, cranberry or a combination thereof and at least one suitable excipient, wherein said peroral composition is a source of constituents selected from the group consisting of 6-(3,4-dihydroxyphenyl)-g-valerolactone, δ-(3-Methoxy-4-hydroxy-phenyl)-γ-valerolactone, catechin, epicatechin, ferulic acid, gallic acid, 4-hydroxybenzoic acid, caffeic acid, protocatechuic acid, taxifolin and mixtures thereof, which are responsible for mediating the anti-inflammatory effects on the endothelium, for use in the treatment of endothelial inflammation and/or endothelial systemic dysfunction triggered by Corona virus disease 2019 (COVID-19) induced by Severe Acute Respiratory Syndrome Corona Virus 2 (SARS-CoV-2) infection.

It is another object to propose a peroral composition comprising procyanidins originated from a plant extract selected from the group consisting in extracts of pine bark, grape seed, apples, cocoa, peanut skin, cranberry or a combination thereof and at least one suitable excipient, wherein said peroral composition is a source of constituents selected from the group consisting of δ-(3,4-dihydroxyphenyl)-g-valerolactone, δ-(3-Methoxy-4-hydroxy-phenyl)-γ-valerolactone, catechin, epicatechin, ferulic acid, gallic acid, 4-hydroxybenzoic acid, caffeic acid, protocatechuic acid, taxifolin and mixtures thereof, which are responsible for mediating the anti-inflammatory effects on the endothelium, for use in the treatment of endothelial inflammation and/or endothelial systemic dysfunction triggered by Corona virus disease 2019 (COVID-19) induced by Severe Acute Respiratory Syndrome Corona Virus 2 (SARS-CoV-2) infection, characterized in that said peroral composition is administered to symptomatic post-COVID-19 subjects recovering from COVID-19.

In another aspect, the present invention provides for a dietary or food supplement, a food preparation, a beverage, a nutraceutical, a medicament comprising the composition of the present invention, for the claimed use.

In a further aspect, the invention provides for a peroral composition comprising a source of constituents selected from the group consisting of δ-(3,4-dihydroxyphenyl)-g-valerolactone, δ-(3-Methoxy-4-hydroxy-phenyl)-γ-valerolactone, catechin, epicatechin, ferulic acid, gallic acid, 4-hydroxybenzoic acid, caffeic acid, protocatechuic acid, taxifolin and mixtures thereof, for use in the treatment of endothelial inflammation and/or endothelial systemic dysfunction triggered by Corona virus disease 2019 (COVID-19) induced by Severe Acute Respiratory Syndrome Corona Virus 2 (SARS-CoV-2) infection, characterized in that said peroral composition is administered to symptomatic post-COVID-19 subjects recovering from COVID-19.

Other objects and advantages of the invention will become apparent to those skilled in the art from a review of the ensuing detailed description, which proceeds with reference to the attendant claims.

### DETAILED DESCRIPTION OF THE INVENTION

In the case of conflict, the present specification, including definitions, will control.

Unless defined otherwise, all technical and scientific terms used herein have the same meaning as is commonly understood by one of skill in art to which the subject matter herein belongs. As used herein, the following definitions are supplied in order to facilitate the understanding of the present invention.

As used in the specification and claims, the singular form "a", "an" and "the" include plural references unless the context clearly dictates otherwise.

The presence of broadening words and phrases such as "one or more," "at least," "but not limited to" or other like phrases in some instances shall not be read to mean that the narrower case is intended or required in instances where such broadening phrases may be absent.

Also, the use of "or" means "and/or" unless otherwise stated.

Similarly, "comprise", "comprises", "comprising", "include", "includes" and "including" are interchangeable and not intended to be limiting. The term "comprise" is generally used in the sense of include, that is to say permitting the presence of one or more features or components.

It is to be further understood that where descriptions of various embodiments use the term "comprising", those skilled in the art would understand that in some specific instances, an embodiment can be alternatively described using language "consisting essentially of" or "consisting of'.

The terms "preferred" and "preferably" refer to embodiments of the disclosure that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the disclosure.

As used herein the term "pine bark extract" refers to a French maritime pine bark extract which is, for example, commercially available as Pycnogenol^{®} (Horphag). The terms "Pycnogenol^{®}", "pine bark extract" and "French maritime pine bark extract" are interchangeable. Pinus pinaster (P. pinaster) and Pinus maritima (P. maritime), are understood to refer to the same organism commonly called "French Maritime Pine." Hence, these terms are interchangeable.

The term "extract", as used herein includes any preparation obtained from plants, fruits or vegetables using an extraction method.

The term "food preparation" refers generally to material of either plant or animal origin, or of synthetic sources, that contain essential nutrients such as a carbohydrate, protein, fat, vitamin, mineral, etc. used in the body of an organism to sustain growth, repair, and vital processes and to furnish energy.

A "dietary or food supplement" refers to a product that contains substances like vitamins, minerals, foods, botanicals, amino acids and is intended to supplement the usual intake of these substances. Dietary supplements are found in pill, tablet, capsule, powder or liquid form and are meant to be taken by mouth.

The term "nutraceutical" refers to any substance that is a food or a part of a food and provides medical or health benefits, including the prevention and treatment of disease. Such products may range from isolated nutrients, dietary supplements and specific diets to genetically engineered designer foods, herbal products, and processed foods such as cereals, soups and beverages. It also refers to a product isolated or purified from foods, and generally sold in medicinal forms not usually associated with food and demonstrated to have a physiological benefit or provide protection against diseases like chronic diseases for example.

The term "beverage" means a liquid for drinking, which may be water, flavored water, soft drinks, alcoholic drink, health drink, or an enriched drink like based on a diary product (milk) or fruit juice.

"Pharmaceutically acceptable excipients or carriers" are any materials that do not interfere with the pharmacological activity of the active ingredient(s) or degrade the body functions of the subject to which it can be administered but facilitate fabrication of dosage forms or administration of the composition. Examples of pharmaceutically acceptable excipient include but are not limited to maltodextrin, calcium phosphate, and fused silica. Pharmaceutically acceptable excipients also include flavorants, as well as various additives such as other vitamins and minerals, all solvents, dispersion media, coatings, isotonic and absorption delaying agents, sweeteners and the like, non-toxic auxiliary substances such as wetting or emulsifying agents, pH buffering agents and the like, such as for example, sodium acetate, sorbitan monolaurate, triethanolamine oleate, and inert ingredients such as talc and magnesium stearate which are standard excipients in the manufacture of tablets, capsules and other dosage forms.

As used herein the terms "subject" or "patient" are well-recognized in the art, and, are used interchangeably herein to refer to a mammal, including dog, cat, rat, mouse, monkey, cow, horse, goat, sheep, pig, camel, and, most preferably, a human. In some embodiments, the subject is a subject in need of treatment or a subject with a disease or disorder. However, in other embodiments, the subject can be a normal subject. The term does not denote a particular age or sex. Thus, adult and newborn subjects, whether male or female, are intended to be covered.

The term "an effective amount" refers to an amount necessary to obtain a physiological effect. The physiological effect may be achieved by one application dose or by repeated applications. The dosage administered may, of course, vary depending upon known factors, such as the physiological characteristics of the particular composition; the age, health and weight of the subject; the nature and extent of the symptoms; the kind of concurrent treatment; the frequency of treatment; and the effect desired and can be adjusted by a person skilled in the art.

Surprisingly it was found that the administration of a composition comprising procyanidins, in a suitable excipient, is particularly promising in the treatment and prevention of endothelial inflammation and/or endothelial systemic dysfunction triggered by Corona virus disease 2019 (COVID-19).

Endothelium is a single layer of squamous endothelial cells that line the interior surface of blood vessels, and lymphatic vessels. The endothelium forms an interface between circulating blood or lymph in the lumen and the rest of the vessel wall. Endothelial cells form the barrier between vessels and tissue and control the flow of substances and fluid into and out of a tissue. Endothelial cells in direct contact with blood are called vascular endothelial cells whereas those in direct contact with lymph are known as lymphatic endothelial cells. Vascular endothelial cells line the entire circulatory system, from the heart to the smallest capillaries. These cells have unique functions that include fluid filtration, such as in the glomerulus of the kidney, blood vessel tone, hemostasis, neutrophil recruitment, and hormone trafficking. Endothelium of the interior surfaces of the heart chambers is called endocardium. An impaired function can lead to serious health issues throughout the body.

"Endothelial inflammation" is an immune response within the endothelium in blood vessels, in which they become inflamed also called endotheliitis. COVID-19 subjects may suffer from an inflammation of all endothelial tissue in a wide range of organs. It has been observed that in COVID-19, endothelial inflammation results in widespread endothelial dysfunction. COVID-19-endotheliitis is responsible for the systemic impaired microcirculatory function in different vascular beds and their clinical sequelae in patients with COVID-19.

Endothelial activation encompasses a range of endothelial responses to inflammatory signals including changes in thromboresistance, altered vasomotor tone, and loss of barrier function. When activated, the endothelium quickly facilitates cellular trafficking. Leukocyte activation and transmigration is crucial for normal innate and adaptive immunity. The term endothelial dysfunction may be applied to states in which the endothelial cell phenotype poses a net liability to the host. The endothelial response to injury can result in vasoconstriction, vasodilatation, vascular leakage, and inflammation. Endothelial activation can transform the internal vascular surface from a non-adhesive barrier into one that recruits leukocytes, is procoagulant, and furthers the inflammatory process.

Vasculitis is a group of disorders that destroy blood vessels by inflammation.

Myocarditis, also known as inflammatory cardiomyopathy, is inflammation of the heart muscle. Both vasculitis and myocarditis have been linked to endothelial activation and have been observed in patients suffering from COVID-19.

"Endothelial dysfunction", or the loss of proper endothelial function, is a hallmark for vascular diseases, and is often regarded as a key early event in the development of atherosclerosis. Impaired endothelial function, causing hypertension and thrombosis, is often seen in patients with coronary artery disease, diabetes

mellitus, hypertension, hypercholesterolemia, as well as in smokers. Endothelial dysfunction has also been shown to be predictive of future adverse cardiovascular events and is also present in inflammatory disease such as rheumatoid arthritis and systemic lupus erythematosus. Endothelial dysfunction is a result of changes in endothelial function. After fat (lipid) accumulation and when stimulated by inflammation, endothelial cells become activated, which is characterized by the expression of molecules such as E-selectin, VCAM-1 and ICAM-1, which stimulate the adhesion of immune cells. Additionally, transcription factors, which are substances which act to increase the production of proteins within cells, become activated; specifically AP-1 and NF-κB, leading to increased expression of cytokines such as IL-1, TNFα and IFNγ, which promotes inflammation. This state of endothelial cells promotes accumulation of lipids and lipoproteins in the intima, leading to atherosclerosis, and the subsequent recruitment of white blood cells and platelets, as well as proliferation of smooth muscle cells, leading to the formation of a fatty streak. The lesions formed in the intima, and persistent inflammation lead to desquamation of endothelium, which disrupts the endothelial barrier, leading to injury and consequent dysfunction.

In vascular diseases, endothelial dysfunction is a systemic pathological state of the endothelium. Along with acting as a semi-permeable membrane, the endothelium is responsible for maintaining vascular tone and regulating oxidative stress by releasing mediators, such as nitric oxide, prostacyclin and endothelin, and controlling local angiotensin-II activity.

Proanthocyanidins designates a group of flavonoids that includes the subgroups procyanidins, prodelphinidins and propelargonidins. Proanthocyanidins are homogeneous or heterogeneous polymers consisting of the monomer units catechin or epicatechin, which are connected either by 4-8 or 4-6 linkages, to the effect that a great number of isomer proanthocyanidins exist. Typically, the proanthocyanidins oligomers have a chain length of 2-12 monomer units. Proanthocyanidins may be synthesized or extracted from a plant material. Non-limiting examples of plant material sources of proanthocyanidins include grape seeds, grape skin, pine barks, ginkgo leaves, cocoa beans, tamarind, tomato, peanut skin, almond, apple, cranberry, blueberry, tea leaves.

Proanthocyanidins represent a group of plant polyphenols found in roots, barks and fruits with an astringent taste. Proanthocyanidins include the subgroups of procyanidins and prodelphinidins. Proanthocyanidins are biopolymers composed of flavan subunits.

Procyanidins are composed of catechin and epicatechin units, also called monomeric procyanidins. Procyanidins are members of the proanthocyanidin (or condensed tannins) class of flavonoids. They are oligomeric compounds, formed from catechin and epicatechin molecules. Proanthocyanidins have also gallic acid in addition to catechin and epicatechin.

Procyanidins, including the lesser bioactive / bioavailable polymers (4 or more catechines), represent a group of condensed flavan-3-ols that can be found in many plants, most notably apples, maritime pine bark, cinnamon, aronia fruit, cocoa beans, grape seed, grape skin, peanut skin and red wines of Vitis vinifera (the common grape). However, bilberry, cranberry, black currant, green tea, black tea, and other plants also contain these flavonoids, as do cocoa beans. Procyanidins can also be isolated from Quercus petraea and Q. robur heartwood (wine barrel oaks). Açaí oil, obtained from the fruit of the açaí palm (Euterpe oleracea), is rich in numerous procyanidin oligomers.

Apples contain on average per serving about eight times the amount of procyanidin found in wine, with some of the highest amounts found in the Red Delicious and Granny Smith varieties.

A well-known product containing procyanidins, which is available in trade as a preparation of a food supplement under the name Pycnogenol^{®}, is an extract of the French maritime pine bark (Pinus pinaster), see also US patents 3,436,407 (MASQUELIER JACQUES); US 5,720,956 (ROHDEWALD, PETER) and US 6,372,266 (SUZUKI NOBUTAKA et al. Horphag Research Ltd.). Pycnogenol^{®} is a standardized bark extract of the French maritime pine Pinus pinaster, Aiton, subspecies Atlantica des Villar. The quality of this extract is specified in the United States Pharmacopeia (USP 28) (Maritime Pine Extract. In: United States Pharmacopeia. Rockville: United States Pharmacopeial Convention, Inc.; 2005. pp 2115- 2116). The extract consists of a concentrate of polyphenols, which are also contained in fruits and vegetables, but, in low concentrations. The polyphenols are composed from flavonoids, especially procyanidins, and phenolic acids. All these constituents possess the ability to inactivate free radicals. Rohdewald P. A review of the French maritime pine bark extract (Pycnogenol®), a herbal medication with a diverse pharmacology. Int J Clin Pharmacol Ther 2002;40(4): 158-168. Between 65-75% of Pycnogenol^{®} are procyanidins comprising of catechin and epicatechin subunits with varying chain lengths (Rohdewald P. A review of the French maritime pine bark extract (Pycnogenol®), an herbal medication with a diverse clinical pharmacology. Int J Clin Pharmacol Ther 2002; 40: 158-168). Other constituents are polyphenolic monomers, phenolic or cinnamic acids and their glycosides (Id.). Pycnogenol^{®} extract is standardized to contain between 65% and 75% procyanidins (70+/- 5% procyanidins) in compliance with USP 28, compounds known for relatively significant antioxidant and anti-inflammatory activity, among other actions (Rohdewald P. "Pycnogenol®, French Maritime Pine Bark extract", Encyclopedia of Dietary Supplements, 2005, pp 545-553).

In one aspect of the present invention there is provided a composition comprising or consisting in procyanidins according to the claims and at least one suitable excipient, for peroral use in the prevention or treatment of endothelial inflammation and/or endothelial systemic dysfunction triggered by Corona virus disease 2019 (COVID-19) induced by Severe Acute Respiratory Syndrome Corona Virus 2 (SARS-CoV-2) infection.

The composition of the present invention contains from comprises from 20 % to 95 % w/w of procyanidins and a suitable excipient q.s.p. (quantity sufficient per 100% of the total volume). Preferably, the composition of the invention comprises about 30 % to 80 % w/w of procyanidins, more preferably about 40% to 80% w/w of procyanidins and even more preferably about 60% w/w to 80% of procyanidins and a suitable excipient q.s.p.

According to a preferred embodiment, the composition of the invention comprises from 65 % to 75 % w/w of procyanidins. Pycnogenol^{®} extract is standardized to contain between 65% and 75% of procyanidins (70+/- 5% procyanidins). Thus, for example tablets of 100 mg of the composition of the invention contain between 65 mg to 75mg of procyanidins.

According to an embodiment of the invention, endothelial inflammation is selected from the group consisting of endotheliitis, myocarditis or vasculitis.

According to a preferred embodiment, said endothelial inflammation consists in severe endotheliitis.

Endothelial tissue is a cell layer that acts as a protective shield in blood vessels and regulates and balances out various processes in the microvessels. The disruption of this regulatory process can, for example, cause circulatory disorders in organs and body tissue, resulting in cellular necrosis and thus to the death of these organs or tissue.

"Endotheliitis" is an immune response within the endothelium in blood vessels, in which they become inflamed. The condition can cause oedema of the surrounding tissue, including the stroma, and can cause irritation and pain.

It was shown that SARS-CoV-2 not only triggers the inflammation of the lungs, which then causes further complications, but is also directly responsible for systemic endotheliitis, an inflammation of all endothelial tissue in the body which affects all vessel beds - in heart, brain, lung and renal vessels as well as vessels in the intestinal tract. The consequences are fatal: this results in severe microcirculatory disturbances that damage the heart, trigger pulmonary embolisms and vascular occlusions in the brain and intestinal tract and can also lead to multiple organ failure and even death. The endothelial tissue of younger patients is usually capable of coping well with the attacks launched by the virus. The situation is different for patients suffering from hypertension, diabetes, heart failure or coronary heart diseases, all of which have one thing in common - their endothelial function is markedly impaired. If patients such as these become infected with SARS-COV-2, they will be particularly at risk, as their already weakened endothelial function will diminish even further, especially during the phase in which the virus reproduces the most.

"Myocarditis", also known as inflammatory cardiomyopathy, is inflammation of the heart muscle. Symptoms can include shortness of breath, chest pain, decreased ability to exercise, and an irregular heartbeat. The duration of problems can vary from hours to months. Complications may include heart failure due to dilated cardiomyopathy or cardiac arrest. Myocarditis is most often due to a viral infection.

"Vasculitis" is a group of disorders that destroy blood vessels by inflammation. Both arteries and veins are affected. Lymphangitis (inflammation of lymphatic vessels) is sometimes considered a type of vasculitis. Vasculitis is primarily caused by leukocyte migration and resultant damage. Although both occur in vasculitis, inflammation of veins (phlebitis) or arteries (arteritis) on their own are separate entities.

According to a particular embodiment of the invention said vasculitis is a Kawasaki-like disease.

"Kawasaki disease" is a syndrome of unknown cause that results in a fever and mainly affects children under 5 years of age. It is a form of vasculitis, where blood vessels become inflamed throughout the body. The fever typically lasts for more than five days and is not affected by usual medications. Other common symptoms include large lymph nodes in the neck, a rash in the genital area, and red eyes, lips, palms, or soles of the feet. Within three weeks of the onset, the skin from the hands and feet may peel, after which recovery typically occurs. In some children, coronary artery aneurysms form in the heart. While the cause is unknown, it may be due to an infection triggering an autoimmune response in those who are genetically predisposed. It does not spread between people. Diagnosis is usually based on a person's signs and symptoms. Other conditions that may present similarly include scarlet fever, juvenile rheumatoid arthritis, and paediatric multisystem inflammatory syndrome associated with COVID-19.

According to Russel M Viner et al. The Lancet, "Kawasaki-like disease: emerging complication during the COVID-19 pandemic" Volume 395, Issue 10239, P1741-1743, June 6, 2020, studies from several countries have confirmed that severe illness and death due to COVID-19 among children are rare, with accurate estimates unavailable because of an absence of true population denominators. However, attention has now shifted to the vulnerability of children for two reasons. First, the degree to which children transmit COVID-19 is key to how countries reopen communities after lockdown. Second, new concerns about a novel severe Kawasaki-like disease in children related to COVID-19, including Lucio Verdoni and colleagues' description of an outbreak in Italy in *The Lancet*, change our understanding of this disease in children. Verdoni and colleagues describe ten cases (seven boys, three girls; aged 7·5 years [SD 3·5]) of a Kawasaki-like disease occurring in Bergamo, Italy, at the peak of the pandemic in the country (Feb 18 to April 20, 2020), a monthly incidence some 30-fold higher than observed for Kawasaki disease across the previous 5 years.

The composition of the invention comprises procyanidins originated from a plant extract or from a synthesized material (i.e., synthetic procyanidins).

The plant extract can be selected from the group consisting of procyanidins containing extracts selected among extracts of pine bark, the cones of cypresses grape seed, apples, peanut skin, walnuts, pomegranates, tomatoes, almonds, tea, hawthorn, cocoa or combination thereof. Procyanidins containing rich extracts are natural and preferably plant extracts having more than 50% by weight (of dried extracts) of procyanidins, more preferably more than 70% by weight and even more preferably more than 75% by weight of procyanidins. Preferably the plant extract according to the present invention is originated from pine bark and more preferably the plant extract is Pycnogenol ^{®}.

In a preferred embodiment, the preparation comprising procyanidins may be a pine bark extract. The pine bark may be from P. pinaster, such as, for example, from Pycnogenol^{®}. In a preferred embodiment, the composition may contain procyanidins at a concentration of 10% to 100% of total weight. For example, a Pycnogenol^{®} composition may be diluted or concentrated to contain 10%, 20%, 30%, 40%, 50%, 60%, 70%, 75%, 80%, 90% or 95% procyanidins. Concentration may be performed using known methods such as column chromatography or affinity chromatography.

According to an embodiment of the invention, the composition comprising procyanidins is a source of constituents selected from the group consisting of 6-(3,4-dihydroxyphenyl)-g-valerolactone, δ-(3-Methoxy-4-hydroxy-phenyl)-γ-valerolactone, catechin, epicatechin, ferulic acid, gallic acid, 4-hydroxybenzoic acid, caffeic acid, protocatechuic acid, taxifolin and mixtures thereof, wherein said constituents are responsible for mediating the anti-inflammatory effects on the endothelium.

Without being bound by theory it is believed that said constituents mediates anti-inflammatory effects by decreasing nitrite production in LPS-stimulated macrophages through direct NO quenching and downregulation of iNOS (inducible NO synthase).

According to Grimm et al. "Single and multiple dose pharmacokinetics of maritime pine bark extract (Pycnogenol) after oral administration to healthy volunteers" BMC Clinical Pharmacology, 03 August 2006, 6:4 - http://www.biomedcentral.com/1472-6904/6/4, a first systematic pharmacokinetic analysis of constituents and metabolites of the standardized maritime pine bark extract (USP quality) after single and repeated intake by human volunteers was carried out. Components of the extract were bioavailable and detectable in the plasma of all subjects. Pharmacokinetic parameter calculated for the so far identified compounds were comparable with results from other studies. In addition, for the first time it was described steady state concentrations of catechin, caffeic acid, ferulic acid and M1 (6-(3,4-dihydroxyphenyl)-γ-valerolactone) and present the first taxifolin plasma concentrations in humans. The detection of ten so far unknown bioavailable constituents and metabolites of Pycnogenol reveal potential for uncovering active compounds with anti-inflammatory bioefficacy.

In accordance with the invention, the composition also comprises at least one suitable excipient, preferably said suitable excipient is a pharmaceutically acceptable excipient.

Examples of suitable excipients of this invention include, but are not limited to, anti-adherents, binders (e.g., macrocrystalline cellulose, gum tragacanth, or gelatin), coatings, disintegrants, fillers, diluents, softeners, emulsifiers, flavoring agents, coloring agents, adjuvants, lubricants, functional agents (e.g., nutrients), viscosity modifiers, bulking agents, glidiants (e.g., colloidal silicon dioxide) surface active agents, osmotic agents, diluents, or any other non-active ingredient, or combinations thereof.

For example, the composition of the present invention may include excipient materials selected from the group consisting of calcium carbonate, coloring agents, whiteners, preservatives, and flavors, triacetin, magnesium stearate, sterotes, natural or artificial flavors, essential oils, plant extracts, fruit essences, gelatins, or combinations thereof.

Optionally the preparation of the present invention may include other artificial or natural sweeteners, bulk sweeteners, or combinations thereof. Bulk sweeteners include both caloric and non-caloric compounds. Non-limiting examples of bulk sweeteners include sucrose, dextrose, maltose, dextrin, dried invert sugar, fructose, high fructose corn syrup, levulose, galactose, corn syrup solids, tagatose, polyols (e.g., sorbitol, mannitol, xylitol, lactitol, erythritol, and maltitol), hydrogenated starch hydrolysates, isomalt, trehalose, and combinations thereof.

Optionally the composition of the present invention can further comprise a non-steroidal anti-inflammatory drug such as acetylsalicylic acid (aspirin).

The composition is adapted for an oral administration.

Preferably, said oral administration is in the form of a food preparation, a dietary supplement, a nutraceutical, or a beverage.

Alternatively, the invention provides for a medicament comprising the composition as defined above.

Thus, the present invention further provides for a food preparation, a dietary or food supplement, a nutraceutical, a beverage, a medicament comprising the composition of the present invention. As described above, the medicament may further comprise acetylsalicylic acid (aspirin) as well as a pharmaceutically acceptable excipient.

Preferably, the dietary supplement, the nutraceutical or the medicament of the present invention is administered at a dosage from or between 25 mg per day to 500 mg per day, more preferably from 25 mg per day to 300 mg per day. The dietary supplement, the nutraceutical or the medicament of the present invention contains from 20 % to 80 % w/w of procyanidins and a suitable excipient q.s.p.

Preferably, the composition of the invention consists in Pycnogenol^{®} extract which is standardized to contain between 65% and 75% of procyanidins. Therefore, tablets of 100 mg may contain between 65 mg and 75mg of procyanidins, more preferably 70 mg of procyanidins,

Said subject in need thereof is an animal, preferably a mammal, and more preferably a human.

Intended for oral administration, the composition or the medicament of the present invention can be in the form, for example, of a tablet, a caplet, a pill, a hard or soft capsule, a lozenge, a cachet, a dispensable powder, granules, a suspension, an elixir, a dispersion, a liquid, or any other form reasonably adapted for such administration.

In a preferred embodiment, the composition according to the invention comprises procyanidins as the sole active ingredients administered to a subject.

Also encompassed is a composition comprising a source of constituents selected from the group consisting of δ-(3,4-dihydroxyphenyl)-g-valerolactone, δ-(3-Methoxy-4-hydroxy-phenyl)-γ-valerolactone, catechin, epicatechin, ferulic acid, gallic acid, 4-hydroxybenzoic acid, caffeic acid, protocatechuic acid, taxifolin and mixtures thereof, for use in the prevention or treatment of endothelial inflammation and/or endothelial systemic dysfunction triggered by Corona virus disease 2019 (COVID-19) induced by Severe Acute Respiratory Syndrome Corona Virus 2 (SARS-CoV-2) infection.

Preferably, said source of constituents is originating from a preparation comprising from 20 % to 95 % w/w of procyanidins. More preferably, said source of constituents is originating from a preparation comprising from 65 % to 75 % w/w of procyanidins.

According to another embodiment, the invention provides for a peroral composition comprising procyanidins originated from a plant extract selected from the group consisting in extracts of pine bark, grape seed, apples, cocoa, peanut skin, cranberry or a combination thereof and at least one suitable excipient, wherein said peroral composition is a source of constituents selected from the group consisting of 6-(3,4-dihydroxyphenyl)-g-valerolactone, δ-(3-Methoxy-4-hydroxy-phenyl)-γ-valerolactone, catechin, epicatechin, ferulic acid, gallic acid, 4-hydroxybenzoic acid, caffeic acid, protocatechuic acid, taxifolin and mixtures thereof, which are responsible for mediating the anti-inflammatory effects on the endothelium, for use in the treatment of endothelial inflammation and/or endothelial systemic dysfunction triggered by Corona virus disease 2019 (COVID-19) induced by Severe Acute Respiratory Syndrome Corona Virus 2 (SARS-CoV-2) infection.

In a preferred embodiment the invention provides for a peroral composition comprising procyanidins originated from a plant extract selected from the group consisting in extracts of pine bark, grape seed, apples, cocoa, peanut skin, cranberry or a combination thereof and at least one suitable excipient, wherein said peroral composition is a source of constituents selected from the group consisting of δ-(3,4-dihydroxyphenyl)-g-valerolactone, δ-(3-Methoxy-4-hydroxy-phenyl)-γ-valerolactone, catechin, epicatechin, ferulic acid, gallic acid, 4-hydroxybenzoic acid, caffeic acid, protocatechuic acid, taxifolin and mixtures thereof, which are responsible for mediating the anti-inflammatory effects on the endothelium, for use in the treatment of endothelial inflammation and/or endothelial systemic dysfunction triggered by Corona virus disease 2019 (COVID-19) induced by Severe Acute Respiratory Syndrome Corona Virus 2 (SARS-CoV-2) infection, characterized in that said peroral composition is administered to symptomatic post-COVID-19 subjects recovering from COVID-19.

Endothelial dysfunction triggered by COVID-19 has been observed to often persist in post-COVID patients even several months after SARS-CoV-2 infection (Riou M, et al. Reduced Flow-Mediated Dilatation Is Not Related to COVID-19 Severity Three Months after Hospitalization for SARS-CoV-2 Infection. Journal of Clinical Medicine. 2021; 10(6):1318. https://doi.org/10.3390/jcm10061318). It has been proposed that endothelial function (using the flow-mediated dilation technique (FMD)) should be evaluated for its value, both as risk stratification and in the early detection of vascular sequelae, as well as for long-term cardiovascular complications in COVID-19 patients (Evans, P.C.; et al. Endothelial Dysfunction in COVID-19: A Position Paper of the ESC Working Group for Atherosclerosis and Vascular Biology, and the ESC Council of Basic Cardiovascular Science. Cardiovasc. Res. 2020).

Accordingly, it was observed that endothelial dysfunction triggered by COVID-19 could still persists several months after SARS-CoV-2 infection in patients recovering from COVID. The peroral composition according to the present invention is thus indifferently suitable for use in the treatment of endothelial inflammation and/or endothelial systemic dysfunction of patients suffering from COVID infection including post-COVID-19 patients or subjects recovering from COVID-19.

Preferably the peroral composition of the invention comprises from 20 % to 95 % w/w of Procyanidins and is administered at a dosage from 25 mg per day to 300 mg per day. More preferably the peroral composition comprises from 65 % to 75 % w/w of procyanidins.

According to one embodiment, the endothelial systemic dysfunction is selected from the group comprising endothelial blood flow and microcirculation or endothelial coagulation function troubles.

In particular the endothelial blood flow and microcirculation function troubles are selected from the group comprising kidney function troubles, lung function troubles, liver function troubles, brain cognition function troubles, endothelial dysfunction related blood pressure troubles endothelial dysfunction blood velocity troubles.

Specifically, the endothelial coagulation function troubles is selected from the group comprising thrombosis, platelet aggregation.

In particular, the endothelial inflammation is selected from the group comprising endotheliitis, myocarditis or vasculitis.

Preferably the vasculitis is a Kawasaki-like disease.

According to another embodiment of the invention, the endotheliitis is a severe endotheliitis. Preferably, the pine bark extract is Pycnogenol ^{®}.

According to one embodiment, the suitable excipient is a pharmaceutically acceptable excipient.

Advantageously, the peroral composition of the invention is in the form of a food preparation, a dietary supplement, a nutraceutical, or a beverage.

According to one embodiment, the invention provides for a medicament comprising the peroral composition of the invention.

Preferably said medicament or dietary supplement of the invention are administered at a dosage from 25 mg per day to 300 mg per day.

An additional object of the invention is to provide a peroral composition comprising a source of constituents selected from the group consisting of δ-(3,4-dihydroxyphenyl)-g-valerolactone, δ-(3-Methoxy-4-hydroxy-phenyl)-γ-valerolactone, catechin, epicatechin, ferulic acid, gallic acid, 4-hydroxybenzoic acid, caffeic acid, protocatechuic acid, taxifolin and mixtures thereof, for use in the treatment of endothelial inflammation and/or endothelial systemic dysfunction triggered by Corona virus disease 2019 (COVID-19) induced by Severe Acute Respiratory Syndrome Corona Virus 2 (SARS-CoV-2) infection.

According to another embodiment, the peroral composition comprising a source of constituents selected from the group consisting of δ-(3,4-dihydroxyphenyl)-g-valerolactone, δ-(3-Methoxy-4-hydroxy-phenyl)-γ-valerolactone, catechin, epicatechin, ferulic acid, gallic acid, 4-hydroxybenzoic acid, caffeic acid, protocatechuic acid, taxifolin and mixtures thereof, is to use in the treatment of endothelial systemic dysfunction triggered by Corona virus disease 2019 (COVID-19) induced by Severe Acute Respiratory Syndrome Corona Virus 2 (SARS-CoV-2) infection, wherein said peroral composition is administered to symptomatic post-COVID-19 subjects recovering from COVID-19.

Preferably said source of constituents is originating from a preparation comprising from 20 % to 95 % w/w of procyanidins.

More preferably, said source of constituents is originating from a preparation comprising from 65 % to 75 % w/w of procyanidins.

In example 1, applicants did evaluate the effects of Pycnogenol^{®} in comparison with controls in improving endothelial function, microcirculation, and the inflammatory marker, IL-6 over 3 weeks in symptomatic subjects with COVID-19.

Two groups of selected subjects were comparable at baseline. The groups progressively improved both with the SM (standard management) and with the SM in combination with the supplement. Patients, supplemented with Pycnogenol^{®} showed significantly better improvement compared to the control group patients. No side effects from the supplementation were observed; tolerability was optimal. The progressive evolution over time was visible in all target measurements.

Flow mediated dilation (FMD) was low in all subjects at inclusion. After 2 weeks, FMD was significantly higher in the Pycnogenol^{®} group in comparison with controls (p<0.05 vs controls) and after 3 weeks it continued to improve in the Pycnogenol^{®} group in comparison with controls(p<0.05 vs controls).

This improvement was also observed in reactive hyperemia measured by skin flux increase (laser Doppler measurements) after release of the occlusive suprasystolic pressure cuff. The difference with controls was already statistically significant after 1 week and continued to increase after 2 weeks and 3 weeks. This confirms not only the improvement of the endothelial function but also of the microcirculation.

Regarding the inflammatory marker IL-6 which was elevated at baseline, it gradually decreased over the 3 weeks. After 3 weeks, IL-6 levels were significantly lower in the Pycnogenol^{®} group compared to controls (p<0.05). The difference with the control group is statistically significant after 1 week already.

In conclusion, Pycnogenol^{®} offers a significant solution for managing key parameters associated with symptomatic COVID-19 syndrome.

In example 2, applicants did evaluate the effects of Pycnogenol^{®} in comparison with controls on symptoms of post-COVID-19 syndrome and in improving endothelial function, microcirculation, inflammatory markers and oxidative stress over 3 months in symptomatic subjects recovering from COVID-19.

Two groups of selected subjects were comparable at baseline. The groups progressively improved both with the SM (standard management) and with the SM in combination with the supplement. Patients, supplemented with Pycnogenol^{®} showed significantly better improvement compared to the control group patients. No side effects from the supplementation were observed; tolerability was optimal. The progressive evolution over time was visible in all target measurements.

Endothelial function, low in all subjects at inclusion was assessed by flow mediated dilation (FMD) and finger reactive hyperemia in the microcirculation (laser Doppler measurements) after the release of an occluding suprasystolic cuff). It was significantly improved in the Pycnogenol^{®} group after one month and after 3 months (p<0.05 vs controls). The rate of ankle swelling (RAS) by strain gauge decreased significantly in the supplemented group (p<0.05) in comparison with controls showing an improvement of the capillary filtration rate. At inclusion, the kidney cortical flow velocity indicated a decrease in perfusion (lower systolic and diastolic flow velocity) in all patients. Kidney cortical flow velocity increased significantly with the supplement (p<0.05) in comparison with controls with improvement in systolic velocity and in diastolic component. High sensitivity CRP (hs-CRP) and Il-6 plasma levels decreased progressively over 3 months with a significant more pronounced decrease in the supplement group (p<0.05). The number of patients with normal plasma IL-6 levels at the end of the study was higher (p<0,05) with the supplement. ESR followed the same pattern with a progressive and a more significant decrease in the supplemented subjects (p<0.02). Oxidative stress decreased significantly in the supplemented group (p<0.05) compared with the control group. Blood pressure and heart rate were normalized in all subjects in the supplement group; systolic pressure was significantly lower in the supplemented group (p<0,05) at the end of the study. All other blood parameters (including platelets and clotting factors) were within normal values at the end of the study.

In conclusion, oral Pycnogenol^{®} administration offered a significant option for managing some of the signs and symptoms associated with post-COVID-19 syndrome. This evaluation offers some rationale for the use of Pycnogenol^{®} in this condition that will have significant importance in the coming years.

The foregoing description will be more fully understood with reference to the following Examples.

### Example 1: Pycnogenol^{®} reduces endothelial and microcirculatory dysfunction and inflammation in subjects with symptomatic coronavirus disease 2019 (COVID-19):

The aim of this controlled study was to evaluate the effects of Pycnogenol^{®} in comparison with controls on endothelial function, microcirculation and the inflammatory marker IL-6 in patients suffering from COVID-19.

### Subjects, Methods

### METHODS:

10 subjects with symptomatic COVID-19 were included the day they consulted the doctor/hospital. One group of 5 followed a usual recovery management while 5 comparable subjects received a supplement of 150 mg Pycnogenol^{®} daily (in 3 doses of 50 mg) in addition to the usual treatment for 4 weeks.

Subjects with COVID-19 between 35-70 years old, with no significant medical history before COVID-19 and willing to participate were included in the registry.

Diagnosis of COVID-19 was performed by detection of SARS-CoV-2 RNA by reverse transcription polymerase chain reaction (RT-PCR), using nasopharynx samples.

Exclusion Criteria were any acute or systemic disease, intake of drugs or other supplementation. In the 3-week registry, the 10 subjects with COVID-19 received either a standard management (SM) or Pycnogenol^{®} in combination with SM. One group of 5 subjects followed a standard recovery management while 5 comparable subjects were supplemented with 150 mg of Pycnogenol^{®} daily (in 3 doses of 50 mg) in addition to standard management. Follow-up was for 3 weeks.

The study parameters of all patients were assessed at baseline, 1 week, 2 weeks and 3 weeks at the end of the registry study.

The supplement study was open and comparative.

### Study endpoints

All study parameters were assessed before 10 am, in a room at constant temperature (20 C°), after 20 minutes of acclimatization.

The following study endpoints were considered:
1. Endothelial function & microcirculation.
   a. Flow-mediated dilatation: Flow-mediated dilatation (FMD) of the brachial artery is an established noninvasive technique to assess endothelial function. The technique was performed like it was described previously (Enseleit F, Sudano I, Periat D, Winnik S, Wolfrum M, Flammer AJ, et al. Effects of Pycnogenol on endothelial function in patients with stable coronary artery disease: a double-blind, randomized, placebo-controlled, cross-over study. Eur Heart J. 2012;33(13): 1589-97.) by measuring brachial artery dilatation after a period of suprasystolic occlusion; artery size was measured before and 1 minute after brachial cuff release using high-resolution ultrasound.
   b. Reactive hyperemia. Reactive hyperemia is a noninvasive assessment of peripheral microvascular function and for assessment of endothelial function. A laser Doppler flowmeter (LDF) noninvasively measure skin flux (a defined LDF unit) in minutes following arterial occlusion. Flux was measured after occlusion, during the same procedure described to evaluate the brachial artery (during the same test and within the same time frame). Finger flux was measured at rest, before occlusion as the average of a one-minute continuous recording. The distal, pulpar laser-Doppler finger flux increase was measured after occlusion as previously described (Freccero C, Holmlund F, Bornmyr S, Castenfors J, Johansson AM, Sundkvist G, et al. Laser Doppler perfusion monitoring of skin blood flow at different depths in finger and arm upon local heating. Microvasc Res. 2003;66(3): 183-9). This flux increase is considered a microcirculatory measure of reactive hyperemia and it is decreased or abolished in patients with severe vascular disease or diabetic microangiopathy. It was measured as skin flux increase after occlusion (% laser Doppler flux increase).
2. Inflammatory marker
   Interleukin-6 plasma levels in pg/mL. Elevated IL-6 levels may indicate an ongoing inflammatory response and could be consistent with a systemic infection, localized infection, or chronic inflammatory disease. IL-6 is considered a nonspecific marker associated with an inflammatory response; it is not diagnostic of any specific disease or disease process (including COVID-19).

The primary assessment during the study was endothelial function and the inflammatory marker IL-6.

### Results

10 subjects with moderate/severe symptomatic COVID-19 were included into the study. Two groups were formed. One group of 5 patients followed the usual management without supplementation (control group) while 5 comparable subjects received Pycnogenol^{®} in addition to the usual management. The two groups were comparable at inclusion. There were no dropouts.

No side effects of supplementation were observed; tolerability was optimal.

At the end of the study all subjects were positive for antibodies against **SARS-CoV-2.**

The results of the assessed parameters of the study are shown in Table 1.

**TABLE 1: Summary including all assessed parameters.**

| *=p<0.05 vs controls. PY=Pycnogenol^{®}. CON=controls. | | | | | |
|---|---|---|---|---|---|
| | | visit 1 | visit 2 | visit 3 | visit 4 |
| | | baseline | 1 week | 2 weeks | 3 weeks |
| 1.Endothelial Function & Microcirculation | | | | | |
| a. FMD [%] | PY | 6.9±0.9 | 7.1±0.7 | 8.46±1.1* | 11.14±1.1* |
| | Con | 7.0±0.84 | 6.7±0.9 | 6.9±0.8 | 7.0±0.7 |
| b. Reactive hyperaemia finger skin flux after occlusion [% increase] | PY | 10.6±1.6 | 13.6±2.0* | 16.4±1.6* | 18.5±1.5* |
| | Con | 10.6±1.4 | 10.8±1.3 | 11.1±1.4 | 12.5±1.4 |

| 2. Inflammatory marker | | | | | |
|---|---|---|---|---|---|
| b. IL-6 [PG/ML] | PY | 9.3±1.7 | 5.1±2.0* | 2.6±0.6* | 1.8±0.7* |
| | CON | 9.2±2.3 | 8.5±2.4 | 7.4±2.3 | 6.6±2.2 |

At the time of inclusion, vascular screening showed no significant vascular problems (plaques, intima-media thickening, aneurysms) in all included subjects. This is important to note as vascular atherosclerotic lesions may alter endothelial function.

### Endothelial function and microcirculation.

Flow mediated dilation (FMD) was low in all subjects at inclusion. After 2 weeks, FMD was significantly higher in the Pycnogenol^{®} group (8.46±1.1%) in comparison with controls (6.9±0.8%) (p<0.05 vs controls) and after 3 weeks it continued to improve (11.14±1.1%) in the Pycnogenol^{®} group in comparison with controls (7.0±0.7%) (p<0.05 vs controls).

This improvement was also observed in reactive hyperemia measured by skin flux increase (laser Doppler measurements) after release of the occlusive suprasystolic pressure cuff. The difference with controls was already statistically significant after 1 week (13.6±2.0% vs 10.8±1.3%) and continued to increase after 2 weeks (16.4±1.6% vs 10.8±1.3%) and 3 weeks (18.5±1.5% vs 12.5±1.4%). This confirms not only the improvement of the endothelial function but also of the microcirculation.

Regarding the inflammatory marker IL-6 which was elevated at baseline, it gradually decreased over the 3 weeks. After 3 weeks, IL-6 levels were significantly lower in the Pycnogenol^{®} group compared to controls (p<0.05). Plasma IL-6 levels decreased drastically in the Pycnogenol^{®} group from 9.3±1.7 to 5.1±2.0 pg/ml after one week and to 1.8±0.7 pg/mL after 3 weeks. The difference with the control group is statistically significant after 1 week already. In the control group, plasma IL-6 levels decreased from 9.2±2.3 to 8.5±2.4 after 1 week and to 6.6±2.2 pg/mL after 3 weeks.

In conclusion, Pycnogenol^{®} offers a significant solution for managing key parameters associated with symptomatic COVID-19 syndrome.

This evaluation offers some interesting rationale for the use of Pycnogenol^{®} in this condition that will have significant importance in the coming years.

### Example 2: Preventive effects of Pycnogenol^{®} on cardiovascular risk factors (including endothelial function) and microcirculation in subjects recovering from coronavirus disease 2019 (COVID-19)

The aim of this controlled study was to evaluate the effects of Pycnogenol^{®} in comparison with controls on symptoms of post-COVID-19 syndrome and in improving endothelial function and microcirculation; inflammatory markers and plasma reactive oxygen metabolites were investigated in this 3-month registry study in symptomatic subjects recovering from COVID-19.

### Subjects, Methods

### METHODS:

Sixty subjects recovering from symptomatic COVID-19 were included. One group of 30 followed a standard recovery management while 30 comparable subjects received a supplement of 150 mg Pycnogenol^{®} daily (in 3 doses of 50 mg) in addition to standard management.

Subjects recovering from COVID-19 between 35-70 years old, with no significant medical history before COVID-19 and willing to participate were included in the registry. No drug treatment was used except for symptomatic and occasional pain treatments, as well as appropriate vitamins and diet.

Subjects were included at least 2 months after viral infection. Diagnosis of COVID-19 was performed by detection of SARS-CoV-2 RNA by reverse transcription polymerase chain reaction (RT-PCR), using nasopharynx samples.

Exclusion Criteria were any acute or systemic disease, intake of drugs or other supplementation. In the 90-day registry, the 60 subjects recovering from diagnosed COVID-19 received either a standard management (SM) or Pycnogenol^{®} in combination with SM. One group of 30 subjects followed a standard recovery management while 30 comparable subjects were supplemented with 150 mg of Pycnogenol^{®} daily (in 3 doses of 50 mg) in addition to standard management. Follow-up was for 3 months.

The study parameters of all patients were assessed at baseline, 2 weeks, 1 month and 3 months at the end of the registry study.

The supplement study was open and comparative.

### Study endpoints

All study parameters were assessed before 10 am, in a room at constant temperature (20 C°), after 20 minutes of acclimatization.

The following study endpoints were considered:
1. Endothelial function & microcirculation.
   a. Flow-mediated dilatation: Flow-mediated dilatation (FMD) of the brachial artery is an established noninvasive technique to assess endothelial function. The technique was performed like it was described previously (Enseleit F, Sudano I, Periat D, Winnik S, Wolfrum M, Flammer AJ, et al. Effects of Pycnogenol on endothelial function in patients with stable coronary artery disease: a double-blind, randomized, placebo-controlled, cross-over study. Eur Heart J. 2012;33(13):1589-97.) by measuring brachial artery dilatation after a period of suprasystolic occlusion; artery size was measured before and 1 minute after brachial cuff release using high-resolution ultrasound.
   b. Reactive hyperemia. Reactive hyperemia is a noninvasive assessment of peripheral microvascular function and for assessment of endothelial function. A laser Doppler flowmeter (LDF) noninvasively measure skin flux (a defined LDF unit) in minutes following arterial occlusion. Flux was measured after occlusion, during the same procedure described to evaluate the brachial artery (during the same test and within the same time frame). Finger flux was measured at rest, before occlusion as the average of a one-minute continuous recording. The distal, pulpar laser-Doppler finger flux increase was measured after occlusion as previously described ( Hu S, Belcaro G, Cornelli U, Luzzi R, Cesarone M, Dugall M, et al. Effects of Pycnogenol(R) on endothelial dysfunction in borderline hypertensive, hyperlipidemic, and hyperglycemic individuals: the borderline study. Int Angiol. 2015;34(1):43-52). This flux increase is considered a microcirculatory measure of reactive hyperemia and it is decreased or abolished in patients with severe vascular disease or diabetic microangiopathy. It was measured as skin flux increase after occlusion (% laser Doppler flux increase).
   c. Rate of ankle swelling (RAS). This test quantifies capillary filtration at the ankle. RAS was measured using a strain gauge plethysmograph (SPG16, Hokanson, USA), with the gauge placed at the at the minimum ankle circumference while the patient is resting supine for 30 minutes. The patient is then asked to move to a standing position. RAS is measured by considering the volume in the supine position and after standing (at 10 and 20 minutes) in mL/min per 100 cm³ of tissue (Belcaro G BA, Hoffman U, Nicolaides AN.. Laser Doppler. Med Orion. 2006).
   d. Kidney cortical flow was measured as flow velocity of the arteries (in cm/sec) with a high -resolution color duplex (Preirus, Hitachi, Japan) (Cesarone MR, De Sanctis MT, Laurora G, Ambrosoli L, Marelli C, Belcaro G. Effects of trandolapril on 24-h ambulatory blood pressure in patients with mild-to-moderate essential hypertension. J Cardiovasc Pharmacol. 1994;23 Suppl 4: S65-72.).
2. Inflammatory markers & oxidative stress
   a. Blood high-sensitivity C-reactive protein (hs-CRP) (25). The standards for hs-CRP level, applied in this study were: lower than 1.0 mg/L (low risk of cardiovascular disease); hs-CRP between 1.0 mg/L and 3.0 mg/L (moderate risk of CVD); hs-CRP level of more than 3.0 mg/L (high risk of CVD).
   b. Interleukin-6 plasma levels in pg/mL. Elevated IL-6 levels may indicate an ongoing inflammatory response and could be consistent with a systemic infection, localized infection, or chronic inflammatory disease. IL-6 is considered a nonspecific marker associated with an inflammatory response; it is not diagnostic of any specific disease or disease process (including COVID-19).
   c. Interleukin-6 (IL-6): proportion of patients with normal IL-6 (value ≤1.8 pg/mL).
   d. Erythrocyte sedimentation rate (ESR) in mm/hr. The plasma ESR has been used as a laboratory test to assess acute phase response to inflammation for a long time. ESR is slightly slower and less sensitive than hs-CRP measurement, however provides further accurate and valuable information on the inflammation status of the patient (Lapić I, Padoan A, Bozzato D, Plebani M. Erythrocyte Sedimentation Rate and C-Reactive Protein in Acute Inflammation. Am J Clin Pathol. 2020;153(1):14-29 ). The norms, applied in this study were as follows: men > 50 years, normal ESR value is less than 20; men < 50 years, normal ESR value is less than 15; women > 50 years, ESR value is less than 30; women < 50 years, normal ESR value is less than 20.
   e. Oxidative stress is assessed by measuring plasma free radicals in a drop of blood taken from the fingertip and expressed in Carr Units (Cesarone MR, Belcaro G, Carratelli M, Cornelli U, De Sanctis MT, Incandela L, et al. A simple test to monitor oxidative stress. Int Angiol. 1999;18(2):127-30 ).
3. Blood pressure (systolic SBP and diastolic DBP) and heart rate (HR).

The primary assessment during the study was endothelial function and microcirculation parameters at each visit. Secondary clinical outcomes - all altered at inclusion - included inflammatory markers such as hs-CRP and IL-6, oxidative stress, blood pressure/heart rate,.

**Statistical analysis.** A number of at least 20 subjects for each group (SM and SM + supplementation) was considered necessary to evaluate differences in target parameters over 12 weeks. All results and data were considered as non-parametric; the Mann-Whitney U-test and the ANOVA were used for the main symptoms/complaints and for the tests.

### Results

60 subjects recovering from symptomatic COVID-19 were included into the study. Two groups were formed. One group of 30 patients followed the standard management (SM, control group) while 30 comparable subjects received Pycnogenol^{®} in addition to SM. The two groups were comparable at inclusion. There were no dropouts.

No side effects of supplementation were observed; tolerability was optimal.

At the end of the study all subjects were positive for antibodies against **SARS-CoV-2.**

The results of the assessed parameters of the study are shown in Table 2.

**TABLE 2: Summary including all assessed parameters.**

| *=p<0.05 vs controls. | PY=Pycnogenol^{®}. | CON= controls. | | | | |
|---|---|---|---|---|---|---|
| | | | | | | |
| | | | | | | |
| *NUMBER* | *PY* | *30(14 F)* | *30* | *30* | | |
| | *CON* | *30(13F)* | *30* | *30* | | |

| | | **VISIT 1** | **VISIT 2** | **VISIT 3** | **VISIT 4** | |
|---|---|---|---|---|---|---|
| | | **BASELINE** | **2 WEEKS** | **1 MONTH** | **3 MONTHS** | |
| **1.ENDOTHELIAL FUNCTION & MICROCIRCULATION** | | | | | | |
| **A. FMD [%]** | PY | 6.5±1.2 | 6.6±1.0 | 12.6±0.9* | 18.8±2.8* | |
| | CON | 7.2±1.0 | 7.3±2,1 | 8.0±0.9 | 8.8±1.4 | |
| **B. REACTIVE HYPEREMIA FINGER SKIN FLUX AFTER OCCLUSION [% INCREASE]** | PY | 11.2±2.0 | 16.0±1.0* | 18.0±0.8* | 24.2±2.3* | |
| | CON | 10.4±2.0 | 11.0±0.9 | 13.0±1.0 | 15.0±1.2 | |
| | | | | | | |
| **C. RAS RATE OF ANKLE SWELLING. [ML/MIN PER 100 CM³ OF TISSUE]** | PY | 2.22±0.01 | | 1.23±0.08* | 1.22±0.02* | |
| | CON | 2.26±0.02 | | 2.02±0.04 | 2.03±0.01 | |
| **D KIDNEY SYSTOLIC CORTICAL FLOW VELOCITY[CM/SEC]** | PY | 20.2±2.0 | 21.0±1.5 | 23.0±1.4* | 23.2±2.2* | |
| | CON | 19.8±1.6 | 19.7±0.7 | 19.6±0.8 | 20.2±1.0 | |
| **DIASTOLIC** | PY | 6.1±1 | 8.0±0.9 | 11.0±0.4* | 14.0±0.9* | |
| **COMPONENT [%]** | CON | 6.4±0.9 | 7.0±0.9 | 8.0±0.8 | 9.2±0.7 | |

| **2. INFLAMMATORY MARKERS & OXIDATIVE STRESS** | | | | | | |
|---|---|---|---|---|---|---|
| **A. PLASMA HS-CRP [MG/L]** | PY | 3.3±0.5 | 3.0±0.6 | 1.2±0.6* | 1.2±0.3* | |
| | CON | 3.2±0.4 | 3.2±0.4 | 2.7±0.5 | 2.4±0.2 | |
| **B. IL-6 [PG/ML]** | PY | 3.0±0.7 | | 1.6±0.5* | 1.2±0.3* | |
| | CON | 2.8±0.4 | | 2.3±0.3 | 2.2±0.8 | |
| **C. PATIENTS WITH NORMAL IL-6 ≤1.8 PG/ML** | PY | 2/30 | | 25/30* | 26/30* | |
| | CON | 3/30 | | 11/30 | 16/30 | |
| **D. ESR [MM/HR]** | PY | 26.6±2.2 | 18.0±1.1 | 13.0±1.0* | 11.0±3.0* | |
| | CON | 27.3±3.0 | 26.0±0.9 | 23.0±1.1 | 19.4±2.2 | |
| **E. OXIDATIVE STRESS [CARR UNITS]** | | PY | 411±16 | 365±11* | 358±9* | 362±8* |
| | **3. SBP [MMHG]** | CON | 418±13 | 399±22 | 384±19 | 387±22 |
| | | PY | 138±3.1 | 135±3.0 | 133±3.0 | 131±2.2* |
| | | CON | 139±2.5 | 138±2.5 | 137±2.2 | 137±2.0 |

The parameters progressively improved in both groups, both with SM and with SM in combination with supplementation for all measured parameters.

At the time of inclusion, vascular screening showed no significant vascular problems (plaques, intima-media thickening, aneurysms) in all included subjects. This is important to note as vascular atherosclerotic lesions may alter endothelial function.

Endothelial function & microcirculation Flow mediated dilation (FMD) was low in all subjects at inclusion. It improved significantly in both groups. After 1 month, FMD was significantly higher in the Pycnogenol^{®} group (12.6±0.9%) in comparison with_controls (8.0±0.9%) (p<0.05 vs controls) and after 3 months it was even higher (18.8±2.8%) in the Pycnogenol^{®} group in comparison with controls (8.8±1.4%) (p<0.05 vs controls).

This improvement was also observed in reactive hyperemia measured by skin flux increase (laser Doppler measurements) after release of the occlusive suprasystolic pressure cuff. The difference with controls was already statistically significant after 2 weeks (16.0±1.0 vs 11.0±09) and continued to increase after 1 month (18.0±0.8% vs 13.0±1.0%) and 3 months (24.2±2.3% vs 15.0±1.2%). This confirms not only the improvement of the endothelial function but also of the microcirculation.

The average rate of ankle swelling (RAS) measured in mL/min per 100 cm³ of tissue, decreased significantly in the supplemented group (p<0.05) in comparison with the controls. The difference with controls was statistically significant after 1 month (1.23±0.08 vs 2.02±0.04) and after 3 months (1.22±0.02 vs 2.03±0.01). This showed a significant improvement of the capillary filtration rate, an important parameter of microcirculation.

Kidney cortical flow velocity at inclusion was low in all patients, indicating a significant decrease in perfusion (lower systolic peak flow and lower diastolic flow velocity components). It increased significantly with the supplement in comparison with controls with a significant improvement of the systolic velocity from 20.2±2.0 to 23.2±2.2 cm/sec for the supplement group versus 19.8±1.6 to 20.2±1.0 cm/sec in the control group. The diastolic component, which is the ratio of diastolic to systolic flow velocity expressed as a percentage, significantly increased by more than two-fold in the Pycnogenol^{®} group (from 6.1±1 to 14±0.9%) compared with controls, where it increased from 6.4±0.9 to 9.2±0.7%.

Regarding inflammatory markers, plasma levels of hs-CRP and IL-6, which were elevated at baseline, gradually decreased over the 3 months. After 3 months, both hs-CRP and IL-6 levels were significantly lower in the Pycnogenol^{®} group compared to controls (p<0.05). The difference was significant after 1 month for hs-CRP where it decreased from 3.3±0.5 to 1.2±0.5mg/L and after 3 months where it remained at 1.2±0.3 mg/L compared to controls where it decreased from 3.2±0.4 to 2.4±0.2 mg/L. Plasma IL-6 levels also decreased drastically in the Pycnogenol^{®} group from 3.0±0.7 to 1.6±0.5 after one month and to 1.2±0.3 pg/mL after 3 months. The difference with the control group is statistically significant at 1 and 3 months. In the control group, plasma IL-6 levels decreased from 2.8±0.4 to 2.3±0.3 after 1 month and to 2.2±0.8 pg/mL after 3 months.

The proportion of patients with IL-6 levels in the normal range (i.e ≤ 1.8 pg/mL) was also higher (p<0,05) with the supplement after 1 and 3 months compared with controls (25/30 and 26/30 vs 11/30 and 16/30).

After 3 months, ESR followed the same pattern with a more progressive and a more significant (p<0.02) decrease in the supplemented subjects (from 26.6±2.2 to 11.0±3.0 mm/hr) compared to the control patients (from 27.3±3.0 to 19.4±2.2 mm/hr).

Plasma oxidative stress was assessed by measurement of plasma free radicals (PFR). The level of PFR expressed in Carr units decreased significantly (p<0.05) in the supplemented group (from 411±16 to 362±8 Carr units) compared with the control group (from 418±13 to 387±22 Carr units), which showed a lower and slower rate of improvement over time.

All other blood parameters (including platelets and clotting factors) were within normal values at the end of the study.

Physiological tests. Blood pressure and heart rate were monitored. They were normalized in all subjects in the supplement group; systolic blood pressure (SBP) was significantly lower (p<0.05) in the supplemented group at the end of the study (from 138±3.1 to 131±2.2 with the supplement vs from 139±2.5 to 137±2.0 in the control group).

### Discussion

The immediate and long-term consequences of COVID-19 are numerous, including neurological symptoms such as loss of smell and taste, headache, anxiety and depression, muscular disorders like weakness and fatigue, forms of vasculitis, renal dysfunction, coagulopathies and even pulmonary fibrosis and are matter of ongoing research.

The clinical situation of post-COVID-19 patients include the usual symptoms of convalescence.

A more severe situation is named "long-COVID" condition with clinically severe symptoms and signs, abnormalities in blood tests, an altered Karnofsky performance scale index, all lasting several months. In these cases, the clinical picture compromises a normal lifestyle and standard activity level. The prevalence of prolonged convalescence of at least 3 months after COVID-19 with one or more persisting COVID-19 symptoms was investigated and strongly varied between 32% and 96%.

The management of the clinical situation is not clearly established yet and there are no real guidelines. Relieve of convalescence symptoms may be a significant problem to address in a short period of time. Specific evaluation methods are still needed and are in development.

Most physicians try to work a therapeutic plan that is primarily related to individual subjects and symptom control.

A high level of inflammation may be present in the recovering patients for a long period of time.

Pycnogenol^{®} is a 'soft', safe, natural anti-inflammatory and anti-oxidative agent studied in several preventive and clinical conditions. This natural agent, with high levels of safety and highly standardized composition is used to control inflammation. Naturally-derived products - when possible - may offer a safe solution to avoid the constant use of drugs with adverse effects, such as NSAIDs or corticosteroids.

Pycnogenol^{®} contributes to these beneficial effects by its proven anti-inflammatory effects on the endothelium. Pycnogenol^{®} consists mainly of procyanidins and small molecules such as catechin, ferulic acid, caffeic acid, and taxifolin. The procyanidins in Pycnogenol^{®} are metabolized by gut bacteria into smaller molecules including metabolite M1 (6-(3,4-dihydroxyphenyl)- γ-valerolactone). These compounds could be detected in the plasma of volunteers, supplemented with Pycnogenol^{®}. The metabolite M1 was found to be selectively incorporated by blood cells and endothelial cells, where it is highly enriched by facilitated uptake, showing anti-inflammatory effects within the cells. As COVID-19 was described as an "endothelial disease", Pycnogenol^{®} was shown to support recovery from a SARS-CoV2 infection by its anti-inflammatory properties, which are directly exerted in the endothelium.

In addition, some of the flavonoids, found in plasma after Pycnogenol^{®} intake were shown to be potential inhibitors of angiotensin-converting enzyme 2 (ACE2), the receptor protein necessary for SARS-CoV-2 infection.

The present study shows that patients, recovering from COVID-19 and supplemented with Pycnogenol^{®} have improved endothelial and microcirculatory function and lower levels of inflammation in the blood compared to the control group.

Based on these various beneficial effects on health, Pycnogenol^{®} is proposed to be used as a valuable tool for physicians in a condition that has no clear or significant solution at the moment.

In conclusion, Pycnogenol^{®} offers a significant solution for managing some of the signs and symptoms associated with post-COVID-19 syndrome.

This evaluation offers some interesting rationale for the use of Pycnogenol^{®} in this condition that will have significant importance in the coming years.

## Claims

1. A peroral composition comprising procyanidins originated from a plant extract selected from the group consisting in extracts of pine bark, grape seed, apples, cocoa, peanut skin, cranberry or a combination thereof and at least one suitable excipient, wherein said peroral composition is a source of constituents selected from the group consisting of δ-(3,4-dihydroxyphenyl)-g-valerolactone, δ-(3-Methoxy-4-hydroxy-phenyl)-γ-valerolactone, catechin, epicatechin, ferulic acid, gallic acid, 4-hydroxybenzoic acid, caffeic acid, protocatechuic acid, taxifolin and mixtures thereof, which are responsible for mediating the anti-inflammatory effects on the endothelium, for a peroral use in the treatment of endothelial inflammation and/or endothelial systemic dysfunction triggered by Corona virus disease 2019 (COVID-19) induced by Severe Acute Respiratory Syndrome Corona Virus 2 (SARS-CoV-2) infection, **characterized in that**, said peroral composition comprises from 20 % to 95 % w/w of procyanidins and is administered at a dosage from 25 mg per day to 300 mg per day.

2. The peroral composition for use according to claim 1, **characterized in that** said peroral composition is administered to symptomatic post-COVID-19 subjects recovering from COVID-19.

3. The peroral composition for use according to any of claims 1-2, wherein said peroral composition comprises from 65 % to 75 % w/w of procyanidins.

4. The peroral composition for use according to any of claims 1-3, wherein said endothelial systemic dysfunction is selected from the group comprising endothelial blood flow and microcirculation or endothelial coagulation function troubles.

5. The peroral composition for use according to claim 4, wherein said endothelial blood flow and microcirculation function troubles, is selected from the group comprising kidney function troubles, lung function troubles, liver function troubles, brain cognition function troubles, endothelial dysfunction related blood pressure troubles endothelial dysfunction blood velocity troubles.

6. The peroral composition for use according to claim 4, wherein said endothelial coagulation function troubles is selected from the group comprising thrombosis, platelet aggregation.

7. The peroral composition for use according to any of claims 1-6, wherein said endothelial inflammation is selected from the group comprising endotheliitis, myocarditis or vasculitis.

8. The peroral composition for use according to claim 7, wherein said vasculitis is a Kawasaki-like disease.

9. The peroral composition for use according to claim 7, wherein said endotheliitis is a severe endotheliitis.

10. The peroral composition for use according to any of any of claims 1 to 9, wherein the pine bark extract is Pycnogenol ^{®}.

11. The peroral composition for use according to any of claims 1 to 10, wherein the suitable excipient is a pharmaceutically acceptable excipient.

12. The peroral composition for use according any of claims 1 to 11, wherein the peroral composition is in the form of a food preparation, a dietary supplement, a nutraceutical, or a beverage.

13. A medicament comprising the peroral composition for use according to any of claims 1 to 11.

14. The medicament of claim 13 or the dietary supplement of claim 12, **characterized in that** said medicament or dietary supplement is administered at a dosage from 25 mg per day to 300 mg per day.

15. A peroral composition comprising a source of constituents selected from the group consisting of δ-(3,4-dihydroxyphenyl)-g-valerolactone, δ-(3-Methoxy-4-hydroxy-phenyl)-γ-valerolactone, catechin, epicatechin, ferulic acid, gallic acid, 4-hydroxybenzoic acid, caffeic acid, protocatechuic acid, taxifolin and mixtures thereof, for a peroral use in the treatment of endothelial inflammation and/or endothelial systemic dysfunction triggered by Corona virus disease 2019 (COVID-19) induced by Severe Acute Respiratory Syndrome Corona Virus 2 (SARS-CoV-2) infection, **characterized in that**, said source of constituents is originating from a preparation comprising from 20 % to 95 % w/w of procyanidins and is administered at a dosage from 25 mg per day to 300 mg per day.

16. The peroral composition for use according to claim 15, **characterized in that** said peroral composition is administered to symptomatic post-COVID-19 subjects recovering from COVID-19.

17. The peroral composition for use according to claims 15 or 16, wherein said source of constituents is originating from a preparation comprising from 65 % to 75 % w/w of procyanidins.

## Patentansprüche

1. Perorale Zusammensetzung, umfassend Procyanidine, die von einem Pflanzenextrakt stammen, das aus der Gruppe ausgewählt ist, die aus Extrakten von Kiefernrinde, Traubenkernen, Äpfeln, Kakao, Erdnusshaut, Cranberry oder einer Kombination davon besteht, und zumindest einen geeigneten Hilfsstoff, wobei die perorale Zusammensetzung eine Quelle von Bestandteilen ist, die aus der Gruppe ausgewählt sind, die aus ö-(3,4-Dihydroxyphenyl)-g-valerolacton, δ-(3-Methoxy-4-hydroxy-phenyl)-γ-valerolacton, Catechin, Epicatechin, Ferulasäure, Gallussäure, 4-Hydroxybenzoesäure, Koffeinsäure, Protocatechusäure, Taxifolin und Gemischen davon besteht, die für das Vermitteln der entzündungshemmenden Wirkungen auf das Endothel verantwortlich sind, zur peroralen Verwendung bei der Behandlung von entdothelialer Entzündung und/oder endothelischer systemischer Dysfunktion, die durch eine Corona-Viruserkrankung 2019 (COVID-19) ausgelöst wurde, die durch eine Infektion mit dem Schwere-akute-Atemnotsyndrom-Coronavirus 2 (SARS-CoV-2) induziert wurde, **dadurch gekennzeichnet, dass** die perorale Zusammensetzung 20 Gew.-% bis 95 Gew.-% Procyanidine umfasst und in einer Dosierung von 25 mg pro Tag bis 300 mg pro Tag verabreicht wird.

2. Perorale Zusammensetzung zur Verwendung nach Anspruch 1, **dadurch gekennzeichnet, dass** die perorale Zusammensetzung symptomatischen Post-COVID-19-Individuen verabreicht wird, die sich von COVID-19 erholen.

3. Perorale Zusammensetzung zur Verwendung nach einem der Ansprüche 1 bis 2, wobei die perorale Zusammensetzung 65 Gew.-% bis 75 Gew.-% an Procyanidinen umfasst.

4. Perorale Zusammensetzung zur Verwendung nach einem der Ansprüche 1 bis 3, wobei die endotheliale systemische Dysfunktion aus der Gruppe ausgewählt ist, die Funktionsprobleme in Bezug auf endothelialen Blutfluss und Mikrozirkulation oder endotheliale Gerinnung umfasst.

5. Perorale Zusammensetzung zur Verwendung nach Anspruch 4, wobei die Funktionsprobleme in Bezug auf endothelialen Blutfluss und Mikrozirkulation aus der Gruppe ausgewählt sind, die Nierenfunktionsprobleme, Lungenfunktionsprobleme, Leberfunktionsprobleme, Probleme mit der kognitiven Funktion des Gehirns, endotheliale Dysfunktion in Zusammenhang mit Blutdruckproblemen, Probleme mit der Blutgeschwindigkeit bei endothelialer Dysfunktion umfasst.

6. Perorale Zusammensetzung zur Verwendung nach Anspruch 4, wobei die Funktionsprobleme in Bezug auf die endotheliale Gerinnung aus der Gruppe ausgewählt sind, die Thrombose, Blutplättchenaggregation umfasst.

7. Perorale Zusammensetzung zur Verwendung nach einem der Ansprüche 1 bis 6, wobei die endotheliale Entzündung aus der Gruppe ausgewählt ist, die Endotheliitis, Myokarditis oder Vaskulitis umfasst.

8. Perorale Zusammensetzung zur Verwendung nach Anspruch 7, wobei die Vaskulitis eine Kawasaki-like-Erkrankung ist.

9. Perorale Zusammensetzung zur Verwendung nach Anspruch 7, wobei die Endotheliitis eine schwere Endotheliitis ist.

10. Perorale Zusammensetzung zur Verwendung nach einem nach einem der Ansprüche 1 bis 9, wobei der Kieferrindenextrakt Pycnogenol^{®} ist.

11. Perorale Zusammensetzung zur Verwendung nach einem der Ansprüche 1 bis 10, wobei der geeignete Hilfsstoff ein pharmazeutisch unbedenklicher Hilfsstoff ist.

12. Perorale Zusammensetzung zur Verwendung nach einem der Ansprüche 1 bis 11, wobei die perorale Zusammensetzung in Form einer Nahrungsmittelzubereitung, eines Nahrungsergänzungsmittels, eines Nutrazeutikums oder eines Getränks vorliegt.

13. Medikament, das die perorale Zusammensetzung zur Verwendung nach einem der Ansprüche 1 bis 11 umfasst.

14. Medikament nach Anspruch 13 oder Nahrungsergänzungsmittel nach Anspruch 12, **dadurch gekennzeichnet, dass** das Medikament oder das Nahrungsergänzungsmittel in einer Dosierung von 25 mg pro Tag bis 300 mg pro Tag verabreicht wird.

15. Perorale Zusammensetzung, die eine Quelle von Bestandteilen umfasst, die aus der Gruppe ausgewählt sind, die aus δ-(3,4-Dihydroxyphenyl)-g-valerolacton, δ-(3-Methoxy-4-hydroxy-phenyl)-γ-valerolacton, Catechin, Epicatechin, Ferulasäure, Gallussäure, 4-Hydroxybenzoesäure, Koffeinsäure, Protocatechusäure, Taxifolin und Gemischen davon besteht, zur peroralen Verwendung bei der Behandlung von endothelialer Entzündung und/oder endothelialer systemischer Dysfunktion, die durch eine Corona-Viruserkrankung 2019 (COVID-19) ausgelöst wurde, die durch eine Infektion mit dem Schwereakute-Atemnotsyndrom-Coronavirus 2 (SARS-CoV-2) induziert wurde, **dadurch gekennzeichnet, dass** die 20 Gew.-% bis 95 Gew.-% Procyanidine umfasst und in einer Dosierung von 25 mg pro Tag bis 300 mg pro Tag verabreicht wird.

16. Perorale Zusammensetzung zur Verwendung nach Anspruch 15, **dadurch gekennzeichnet, dass** die perorale Zusammensetzung symptomatischen Post-COVID-19-Individuen verabreicht wird, die sich von COVID-19 erholen.

17. Perorale Zusammensetzung zur Verwendung nach Anspruch 15 oder 16, wobei die Quelle von Bestandteilen aus einer Zubereitung stammt, die 65 Gew.-% bis 75 Gew.-% an Procyanidinen umfasst.

## Revendications

1. Composition orale comprenant des procyanidines issues d'un extrait végétal choisi dans le groupe constitué par les extraits d'écorce de pin, de pépins de raisin, de pommes, de cacao, de peau de cacahuète, de canneberge ou d'une de leurs combinaisons, et au moins un excipient convenable, laquelle composition à usage oral est une source de constituants choisis dans le groupe constitué par la δ-(3,4-dihydroxyphényl)-g-valérolactone, la δ-(3-méthoxy-4-hydroxyphényl)-γ-valérolactone, la catéchine, l'épicatéchine, l'acide férulique, l'acide gallique, l'acide 4-hydroxybenzoïque, l'acide caféique, l'acide protocatéchuique, la taxifoline et leurs mélanges, qui sont responsables de la médiation des effets anti-inflammatoires sur l'endothélium, pour une utilisation par voie orale dans le traitement d'une inflammation endothéliale et/ou d'un dysfonctionnement systémique endothélial déclenchés par la maladie à coronavirus 2019 (COVID-19) induite par une infection par le coronavirus 2 du syndrome respiratoire aigu sévère (SARS-CoV-2), **caractérisée en ce que** ladite composition orale comprend 20 % à 95 % en poids/poids de procyanidines et est administrée à une dose de 25 mg par jour à 300 mg par jour.

2. La composition orale pour une utilisation selon la revendication 1, **caractérisée en ce que** ladite composition à usage oral est administrée à des sujets post-COVID-19 symptomatiques en convalescence de la COVID-19.

3. La composition orale pour une utilisation selon l'une quelconque des revendications 1 et 2, où ladite composition à usage oral comprend 65 % à 75 % en poids/poids de procyanidines.

4. La composition orale pour une utilisation selon l'une quelconque des revendications 1 à 3, dans laquelle ledit dysfonctionnement systémique endothélial est choisi dans le groupe comprenant les troubles de la fonction de circulation et de microcirculation sanguine endothéliale ou de coagulation endothéliale.

5. La composition orale pour une utilisation selon la revendication 4, dans laquelle lesdits troubles de la fonction de circulation et de microcirculation sanguine endothéliale sont choisis dans le groupe comprenant les troubles de la fonction rénale, les troubles de la fonction pulmonaire, les troubles de la fonction hépatique, les troubles de la fonction cognitive cérébrale, les troubles de la pression sanguine associés à un dysfonctionnement endothélial, les troubles de la vitesse du sang associés à un dysfonctionnement endothélial.

6. La composition orale pour une utilisation selon la revendication 4, dans laquelle lesdits troubles de la fonction de coagulation endothéliale sont choisis dans le groupe comprenant une thrombose, une agrégation plaquettaire.

7. La composition orale pour une utilisation selon l'une quelconque des revendications 1 à 6, dans laquelle ladite inflammation endothéliale est choisie dans le groupe comprenant une endothéliite, une myocardite et une angéite.

8. La composition orale pour une utilisation selon la revendication 7, dans laquelle ladite angéite est une maladie de type Kawasaki.

9. La composition orale pour une utilisation selon la revendication 7, dans laquelle ladite endothéliite est une endothéliite sévère.

10. La composition orale pour une utilisation selon l'une quelconque des revendications 1 à 9, dans laquelle l'extrait d'écorce de pin est le Pycnogenol^{®}.

11. La composition orale pour une utilisation selon l'une quelconque des revendications 1 à 10, dans laquelle l'excipient convenable est un excipient pharmaceutiquement acceptable.

12. La composition orale pour une utilisation selon l'une quelconque des revendications 1 à 11, où la composition à usage oral est sous la forme d'une préparation alimentaire, d'un complément alimentaire, d'un nutraceutique, ou d'une boisson.

13. Médicament comprenant la composition orale pour une utilisation selon l'une quelconque des revendications 1 à 11.

14. Médicament selon la revendication 13 ou complément alimentaire selon la revendication 12, **caractérisé en ce que** ledit médicament ou complément alimentaire est administré à une dose de 25 mg par jour à 300 mg par jour.

15. Composition orale comprenant une source de constituants choisis dans le groupe constitué par la δ-(3,4-dihydroxyphényl)-g-valérolactone, la δ-(3-méthoxy-4-hydroxyphényl)-γ-valérolactone, la catéchine, l'épicatéchine, l'acide férulique, l'acide gallique, l'acide 4-hydroxybenzoïque, l'acide caféique, l'acide protocatéchuique, la taxifoline et leurs mélanges, pour une utilisation par voie orale dans le traitement d'une inflammation endothéliale et/ou d'un dysfonctionnement systémique endothélial déclenchés par la maladie à coronavirus 2019 (COVID-19) induite par une infection par le coronavirus 2 du syndrome respiratoire aigu sévère (SARS-CoV-2), **caractérisée en ce que** ladite source de constituants est issue d'une préparation comprenant 20 % à 95 % en poids/poids de procyanidines et est administrée à une dose de 25 mg par jour à 300 mg par jour.

16. La composition orale pour une utilisation selon la revendication 15, **caractérisée en ce que** ladite composition orale est administrée à des sujets post-COVID-19 symptomatiques en convalescence de la COVID-19.

17. La composition orale pour une utilisation selon la revendication 15 ou 16, dans laquelle ladite source de constituants est issue d'une préparation comprenant 65 % à 75 % en poids/poids de procyanidines.
